# EUROPEAN PATENT SPECIFICATION

(11) **EP 3 328 877 B1**
(45) Date of publication and mention of the grant of the patent: **24.08.2022**
(21) Application number: 16747567.2
(22) Date of filing: 27.07.2016
(51) Int. Cl.: C07K 7/06, C07K 5/09, C07K 7/64, C07K 1/06

(54) **PEPTOID**
PEPTOID
PEPTOÏDE

(30) Priority: 30.07.2015 GB 201513444
(43) Date of publication of application: 06.06.2018
(73) Proprietor: The University of Durham, Durham DH1 3HP (GB)
(72) Inventor: COBB, Steven, Durham DH1 3LE (GB); BOLT, Hannah, Durham DH1 3LE (GB)
(74) Representative: Hutter, Anton
(86) International application number: PCT/GB2016/052296
(87) International publication number: WO 2017/017445

(56) References cited:
- WO-A1-2009/134942
- WO-A2-2010/098843
- WO-A2-2010/098843
- US-A1- 2014 274 916
- CINDY W WU ET AL: "Helical Peptoid Mimics of Lung Surfactant Protein C", CHEMISTRY AND BIOLOGY., vol. 10, no. 11, 1 November 2003 (2003-11-01), pages 1057-1063, XP55578497, GB ISSN: 1074-5521, DOI: 10.1016/j.chembiol.2003.10.008
- JOHN A. W. KRUIJTZER ET AL: "Peptoid-Peptide Hybrids as Potent Novel Melanocortin Receptor Ligands", JOURNAL OF MEDICINAL CHEMISTRY, vol. 48, no. 13, 1 June 2005 (2005-06-01), pages 4224-4230, XP55578500, US ISSN: 0022-2623, DOI: 10.1021/jm0490033
- THOMAS KLIMKAIT ET AL: "Rational optimization of a HIV-1 Tat inhibitor: Rapid progress on combinatorial lead structures", BIOTECHNOLOGY AND BIOENGINEERING, vol. 61, no. 3, 1 January 1999 (1999-01-01), pages 155-168, XP55578502, ISSN: 0006-3592, DOI: 10.1002/(SICI)1097-0290(1998)61:3<155::AID -CC3>3.0.CO;2-G
- MARINA GOBBO ET AL: "Substitution of the Arginine/Leucine Residues in Apidaecin Ib with Peptoid Residues: Effect on Antimicrobial Activity, Cellular Uptake, and Proteolytic Degradation", JOURNAL OF MEDICINAL CHEMISTRY, vol. 52, no. 16, 27 August 2009 (2009-08-27), pages 5197-5206, XP055036474, ISSN: 0022-2623, DOI: 10.1021/jm900396a
- CHRISTIAN A. OLSEN ET AL: "Antimicrobial, Hemolytic, and Cytotoxic Activities of [beta]-Peptoid-Peptide Hybrid Oligomers: Improved Properties Compared to Natural AMPs", CHEMBIOCHEM - A EUROPEAN JOURNAL OF CHEMICAL BIOLOGY., vol. 11, no. 10, 5 July 2010 (2010-07-05), pages 1356-1360, XP055303637, DE ISSN: 1439-4227, DOI: 10.1002/cbic.201000232
- SEURYNCK-SERVOSS, SHANNON L. ET AL.: "Effects of inducing an N-terminal insertion region and arginine-mimetic side chains in helical peptoid analogues of lung surfactant protein B", BIOCHEMISTRY, vol. 45, no. 39, 2006, pages 11809-11818, XP055017018, cited in the application
- H. L. BOLT ET AL: "A practical method for the synthesis of peptoids containing both lysine-type and arginine-type monomers", ORGANIC & BIOMOLECULAR CHEMISTRY, vol. 14, no. 4, 8 December 2015 (2015-12-08), pages 1211-1215, XP055303619, GB ISSN: 1477-0520, DOI: 10.1039/C5OB02279G
- CINDY W WU ET AL: "Helical Peptoid Mimics of Lung Surfactant Protein C", CHEMISTRY AND BIOLOGY., vol. 10, no. 11, 1 November 2003 (2003-11-01), pages 1057-1063, XP055578497, GB ISSN: 1074-5521, DOI: 10.1016/j.chembiol.2003.10.008
- JOHN A. W. KRUIJTZER ET AL: "Peptoid-Peptide Hybrids as Potent Novel Melanocortin Receptor Ligands", JOURNAL OF MEDICINAL CHEMISTRY, vol. 48, no. 13, 1 June 2005 (2005-06-01), pages 4224-4230, XP055578500, US ISSN: 0022-2623, DOI: 10.1021/jm0490033
- THOMAS KLIMKAIT ET AL: "Rational optimization of a HIV-1 Tat inhibitor: Rapid progress on combinatorial lead structures", BIOTECHNOLOGY AND BIOENGINEERING, vol. 61, no. 3, 1 January 1999 (1999-01-01), pages 155-168, XP055578502, ISSN: 0006-3592, DOI: 10.1002/(SICI)1097-0290(1998)61:3<155::AID -CC3>3.0.CO;2-G

### SEQUENCE LISTING

<110> The University of Durham
<120> PEPTOID
<130> 75568PCT1
<160> 4
<170> PatentIn version 3.5
<210> 1
   <211> 12
   <212> PRT
   <213> Artificial Sequence
<220>
   <221> misc_feature
   <222> (1)..(12)
   <223> Peptide sequence of Peptoid 1, which comprises aritificial amino
   acids whose side chains are bonded to the nitrogen atom of the peptide
backbone
<220>
   <221> MISC_FEATURE
   <222> (1)..(1)
   <223> Xaa is N-(4-aminobutyl) glycine [NLys]
<220>
   <221> MISC_FEATURE
   <222> (2)..(3)
   <223> Xaa is (S)-N-(1-phenylethyl) glycine [Nspe]
<220>
   <221> MISC_FEATURE
   <222> (4)..(4)
   <223> Xaa is N-(4-aminobutyl) glycine [NLys]
<220>
   <221> MISC_FEATURE
   <222> (5)..(6)
   <223> Xaa is (S)-N-(1-phenylethyl) glycine [Nspe]
<220>
   <221> MISC_FEATURE
   <222> (7)..(7)
   <223> Xaa is *N*-(4-guanidinobutyl) glycine [NhArg]
<220>
   <221> MISC_FEATURE
   <222> (8)..(9)
   <223> Xaa is (S)-N-(1-phenylethyl) glycine [Nspe]
<220>
   <221> MISC_FEATURE
   <222> (10)..(10)
   <223> Xaa is *N*-(4-guanidinobutyl) glycine [NhArg]
<220>
   <221> MISC_FEATURE
   <222> (11)..(12)
   <223> Xaa is (S)-N-(1-phenylethyl) glycine [Nspe]
<400> 1
   Xaa Xaa Xaa Xaa Xaa Xaa Xaa Xaa Xaa Xaa Xaa Xaa
   1               5                   10
<210> 2
   <211> 12
   <212> PRT
   <213> Artificial Sequence
<220>
   <221> MISC_FEATURE
   <222> (1)..(12)
   <223> Peptide sequence of Peptoid 2, which comprises aritificial amino
   acids whose side chains are bonded to the nitrogen atom of the peptide
backbone
<220>
   <221> MISC_FEATURE
   <222> (1)..(1)
   <223> Xaa is N-(4-guanidinobutyl) glycine [NhArg]
<220>
   <221> MISC_FEATURE
   <222> (2)..(3)
   <223> Xaa is (S)-N-(1-phenylethyl) glycine [Nspe]
<220>
   <221> MISC_FEATURE
   <222> (4)..(4)
   <223> Xaa is N-(4-guanidinobutyl) glycine [NhArg]
<220>
   <221> MISC_FEATURE
   <222> (5)..(6)
   <223> Xaa is (S)-N-(1-phenylethyl) glycine [Nspe]
<220>
   <221> MISC_FEATURE
   <222> (7)..(7)
   <223> Xaa is N-(4-aminobutyl) glycine [NLys]
<220>
   <221> MISC_FEATURE
   <222> (8)..(9)
   <223> Xaa is (S)-N-(1-phenylethyl) glycine [Nspe]
<220>
   <221> MISC_FEATURE
   <222> (10)..(10)
   <223> Xaa is N-(4-aminobutyl) glycine [NLys]
<220>
   <221> MISC_FEATURE
   <222> (11)..(12)
   <223> Xaa is (S)-N-(1-phenylethyl) glycine [Nspe]
<400> 2
   Xaa Xaa Xaa Xaa Xaa Xaa Xaa Xaa Xaa Xaa Xaa Xaa
   1               5                   10
<210> 3
   <211> 12
   <212> PRT
   <213> Artificial Sequence
<220>
   <221> MISC_FEATURE
   <222> (1)..(12)
   <223> Peptide sequence of Peptoid 3, which comprises aritificial amino
   acids whose side chains are bonded to the nitrogen atom of the peptide
backbone
<220>
   <221> MISC_FEATURE
   <222> (1)..(1)
   <223> Xaa is N-(4-aminobutyl) glycine [NLys]
<220>
   <221> MISC_FEATURE
   <222> (2)..(3)
   <223> Xaa is (S)-N-(1-phenylethyl) glycine [Nspe]
<220>
   <221> MISC_FEATURE
   <222> (4)..(4)
   <223> Xaa is N-(4-guanidinobutyl) glycine [NhArg]
<220>
   <221> MISC_FEATURE
   <222> (5)..(6)
   <223> Xaa is (S)-N-(1-phenylethyl) glycine [Nspe]
<220>
   <221> MISC_FEATURE
   <222> (7)..(7)
   <223> Xaa is N-(4-aminobutyl) glycine [NLys]
<220>
   <221> MISC_FEATURE
   <222> (8)..(9)
   <223> Xaa is (S)-N-(1-phenylethyl) glycine [Nspe]
<220>
   <221> MISC_FEATURE
   <222> (10)..(10)
   <223> Xaa is N-(4-aminobutyl) glycine [NLys]
<220>
   <221> MISC_FEATURE
   <222> (11)..(12)
   <223> Xaa is (S)-N-(1-phenylethyl) glycine [Nspe]
<400> 3
   Xaa Xaa Xaa Xaa Xaa Xaa Xaa Xaa Xaa Xaa Xaa Xaa
   1               5                   10
<210> 4
   <211> 12
   <212> PRT
   <213> Artificial Sequence
<220>
   <221> MISC_FEATURE
   <222> (1)..(12)
   <223> Peptide sequence of Peptoid 4, which comprises aritificial amino
   acids whose side chains are bonded to the nitrogen atom of the peptide
backbone
<220>
   <221> MISC_FEATURE
   <222> (1)..(1)
   <223> Xaa is N-(4-guanidinobutyl) glycine [NhArg]
<220>
   <221> MISC_FEATURE
   <222> (2)..(3)
   <223> Xaa is (S)-N-(1-phenylethyl) glycine [Nspe]
<220>
   <221> MISC_FEATURE
   <222> (4)..(4)
   <223> Xaa is N-(4-aminobutyl) glycine [NLys]
<220>
   <221> MISC_FEATURE
   <222> (5)..(6)
   <223> Xaa is (S)-N-(1-phenylethyl) glycine [Nspe]
<220>
   <221> MISC_FEATURE
   <222> (7)..(7)
   <223> Xaa is N-(4-guanidinobutyl) glycine [NhArg]
<220>
   <221> MISC_FEATURE
   <222> (8)..(9)
   <223> Xaa is (S)-N-(1-phenylethyl) glycine [Nspe]
<220>
   <221> MISC_FEATURE
   <222> (10)..(10)
   <223> Xaa is N-(4-aminobutyl) glycine [NLys]
<220>
   <221> MISC_FEATURE
   <222> (11)..(12)
   <223> Xaa is (S)-N-(1-phenylethyl) glycine [Nspe]
<400> 4
   Xaa Xaa Xaa Xaa Xaa Xaa Xaa Xaa Xaa Xaa Xaa Xaa
   1               5                   10
<210> 5
   <211> 12
   <212> PRT
   <213> Artificial Sequence
<220>
   <221> MISC_FEATURE
   <222> (1)..(12)
   <223> Peptide sequence of Peptoid 5, which is a cyclic peptoid comprising
   aritificial amino acids whose side chains are bonded to the nitrogen atom of
   the peptide backbone
<220>
   <221> MISC_FEATURE
   <222> (1)..(1)
   <223> Xaa is N-(4-aminobutyl) glycine [NLys]
<220>
   <221> MISC_FEATURE
   <222> (2).. (2)
   <223> Xaa is N-(phenylmethyl) glycine [Nphe]
<220>
   <221> MISC_FEATURE
   <222> (3)..(3)
   <223> Xaa is N-(4-guanidinobutyl) glycine [NhArg]
<220>
   <221> MISC_FEATURE
   <222> (4)..(4)
   <223> Xaa is N-(phenylmethyl) glycine [Nphe]
<220>
   <221> MISC_FEATURE
   <222> (5)..(5)
   <223> Xaa is N-(4-aminobutyl) glycine [NLys]
<220>
   <221> MISC_FEATURE
   <222> (6)..(6)
   <223> Xaa is N-(phenylmethyl) glycine [Nphe]
<400> 5
<210> 6
   <211> 12
   <212> PRT
   <213> Artificial Sequence
<220>
   <221> MISC_FEATURE
   <222> (1)..(12)
   <223> Peptide sequence of Peptoid 6, which comprises aritificial amino
   acids whose side chains are bonded to the nitrogen atom of the peptide
backbone
<220>
   <221> MISC_FEATURE
   <222> (1)..(1)
   <223> Xaa is N-(2-guanidinoethyl) glycine [NnArg]
<220>
   <221> MISC_FEATURE
   <222> (2)..(3)
   <223> Xaa is (S)-N-(1-phenylethyl) glycine [Nspe]
<220>
   <221> MISC_FEATURE
   <222> (4)..(4)
   <223> Xaa is N-(2-aminoethyl) glycine [Nae]
<220>
   <221> MISC_FEATURE
   <222> (5)..(6)
   <223> Xaa is (S)-N-(1-phenylethyl) glycine [Nspe]
<220>
   <221> MISC_FEATURE
   <222> (7)..(7)
   <223> Xaa is N-(2-guanidinoethyl) glycine [NnArg]
<220>
   <221> MISC_FEATURE
   <222> (8)..(9)
   <223> Xaa is (S)-N-(1-phenylethyl) glycine [Nspe]
<220>
   <221> MISC_FEATURE
   <222> (10)..(10)
   <223> Xaa is N-(2-aminoethyl) glycine [Nae]
<220>
   <221> MISC_FEATURE
   <222> (11)..(12)
   <223> Xaa is (S)-N-(1-phenylethyl) glycine [Nspe]
<400> 6
   Xaa Xaa Xaa Xaa Xaa Xaa Xaa Xaa Xaa Xaa Xaa Xaa
   1               5                   10

## Description

The present invention relates to peptoids, derivatives and analogues thereof, and to methods of chemically synthesising such compounds. More specifically, the present invention relates to mixed peptoids, derivatives and analogues thereof comprising lysine and arginine type monomers, which may be linear or cyclic, and to their uses in therapy, for example as antimicrobial agents, and in methods for treating microbial (e.g. bacterial) infections.

Since the discovery of penicillin in 1928, a large range of antibiotics have been successfully developed to combat a wide variety of infections. However, resistance to these antibiotics is increasing at a remarkable rate and is becoming a serious problem, with drug resistant strains of previously treatable illnesses on the rise. Current structural classes of antibiotic compounds are becoming redundant and it is widely agreed that there is a desperate need to design, make and test new antibiotic compounds.

Peptides have shown considerable promise as medicines, and investment in this area by the pharmaceutical industry continues to increase. However, many peptide drugs are readily broken down in the human body, which presents drug formulation and delivery challenges. Moreover, physical properties of peptides, such as water-solubility and membrane-permeability, remain highly problematic. Many drugs fail in development at the pre-clinical stage due to poor physical properties and many limitations remain in developing peptide-based drugs with suitable pharmaceutical properties, e.g. membrane permeability, bioavailability and water solubility. Aqueous formulation of peptides can, therefore, be non-trivial and are often formulated with excipients, surfactants and co-solvents, which may result in adverse side effects. Therefore, there is a need to improve peptidic drugs due to their inherently unfavourable pharmacokinetic properties, e.g. stability, membrane permeability, bioavailability and water solubility.

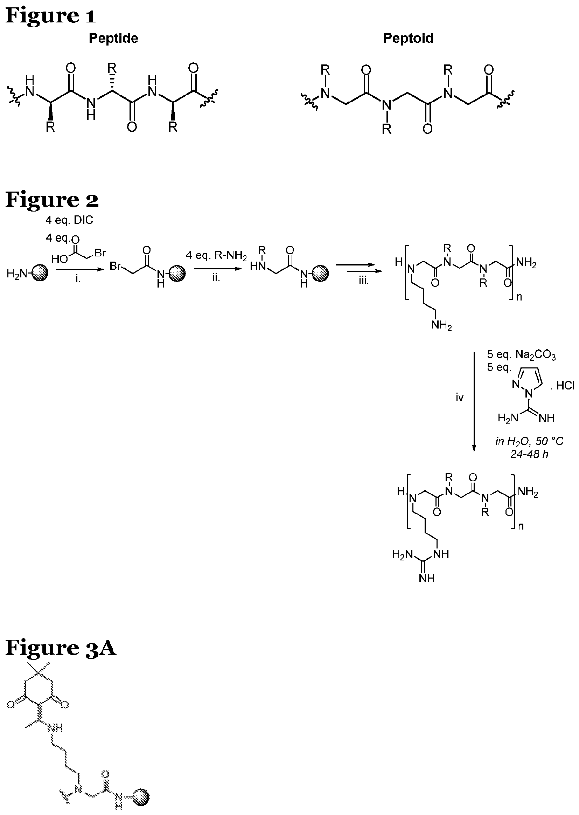

In the development of more stable peptide drugs areas of significant interest lie in the use of stabilised or stapled α-helices, (multi)cyclic peptides and peptidomimetics, which include 'peptoids'. Peptoids, or poly-*N*-substituted glycines, are a class of peptidomimetics whose side chains are appended to the nitrogen atom of the peptide backbone, rather than to the alpha-carbons, as they are in amino acids, and this is shown in Figure 1. Peptoids are an emerging class of therapeutic agent, which are structurally very similar to peptides, but have a superior proteolytic stability in *vivo* when compared with standard peptide-based drugs.

Commonly, lysine-containing peptoids are seen in the literature. Some research groups have sought to improve the biological activity of peptoids by replacing these 'lysine' residues with the 'arginine' analogue (a primary amine to a guanidinium group) since arginine-rich cell-penetrating peptides have been shown to have a high potential to deliver drugs into cultured cells. Guanidine-containing peptoids have been shown to translocate into the cell quicker than amino containing peptoids. [P. A. Wender, D. J. Mitchell, K. Pattabiraman et al., Proc. Natl. Acad. Sci. USA, 2000, 97, 13003-13008; M. L. Huang, S. B. Y. Shin, M. A. Benson et al., ChemMedChem, 2012, 7,114-122.]

Previously synthesised polyarginine-based peptoids were made using the method developed by Rothbard and co-workers using pyrazole-i-carboxamide to modify lysine type side-chains after peptoid synthesis and cleavage from the resin, and shown in Figure 2. [P. A. Wender, D. J. Mitchell, K. Pattabiraman et al., Proc. Natl. Acad. Sci. USA, 2000, 97, 13003-13008]. The disadvantage of this procedure however is that every lysine-type chain is transformed into an arginine-type chain and so mixed peptoid sequences comprising both lysines and arginines cannot be made. This approach only makes arginine side chain peptoids, and mixed Arg/Lys peptoids cannot be accessed using this approach.

Currently there are challenges in balancing the biological activity and the toxicity of peptoid compounds. The inventors believe that the ability to alter the chemical functionality of the cationic side chains in a given peptoid sequence, such as the lysine and arginine type monomers, may assist in these endeavours.

The Zuckermann group (http://www.ronznet.com/index.html) has previously described the synthesis of a PMC-protected guanidinopropyl amine that was suggested to be compatible with the submonomer synthesis of peptoids on resin (T. Uno, E. Beausoleil, R. A. Goldsmith et al., Tetrahedron Lett., 1999, 40, 1475-1478). Barron *et al*., attempted the synthesis of a mixed Arg/Lys peptoid using these PMC-protected amines, however, their poor solubility and the extended cleavage times necessary caused acid-induced degradation of the mixed peptoids and prevented isolation of the required targets (S. L. Seurynck-Servoss, M. T. Dohm and A. E. Barron, Biochem., 2006, 45, 11809-11818). It was only possible to prepare and isolate one mixed Arg/Lys peptoid which was linear and contained no aromatic residues.

Aromatic residues/side chains (particularly α-chiral aromatic residues) have been shown to stabilise a helical secondary structure, which can be important for the biological or materials applications of peptoids. Additionally, being able to include aromatic residues vastly increases the possible sequence variety.

Gobbo et al. ("Substitution of the Arginine/Leucine Residues in Apidaecin Ib with Peptoid Residues: effect on Antimicrobial Activity, Cellular Uptake, and Proteolytic Degradation", J. Med. Chem. 2009, 52, 5197-5206) investigates the effect induced in the insect antimicrobial peptide apidaecin Ib by replacement of a single arginine/leucine residue with a N-substituted glycine.

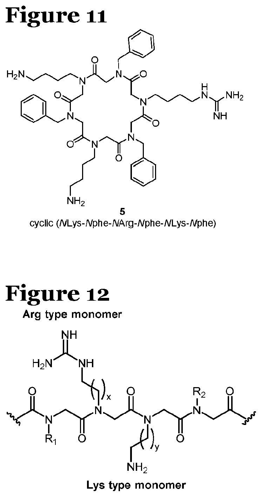

It would be advantageous to be able to be able to prepare mixed Arg/Lys type peptoids (i.e. peptoids that contain amine and guanidine functionalities on their side chains, similar to the generic peptoid structure shown in Figure 12) that were cyclic and/or contain aromatic side chains.

The current invention arises from the inventors' work in trying to overcome the problems associated with the prior art.

In accordance with a first aspect of the invention, there is provided a method of preparing a peptoid, analogue or derivative thereof, comprising at least one arginine type monomer and at least one lysine type monomer, wherein the arginine type monomer comprises a monomer of Formula (I): 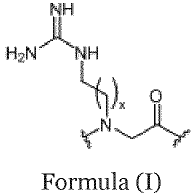 wherein x is an integer between 0 and 14; and
the lysine type monomer comprises a monomer of Formula (II): 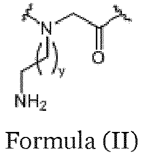 wherein y is an integer between 0 and 14;
the method comprising:-
(i) synthesising a precursor linear peptoid, analogue or derivative thereof comprising one or more lysine type monomers protected with a first protecting group, and one or more lysine type monomers protected with a second protecting group, wherein the first protecting group comprises an N-(1-(4,4-dimethyl-2,6-dioxocyclohexylidene)ethyl) (Dde) protecting group and the second protecting group comprises a tert-butyloxycarbonyl (Boc) protecting group;
(ii) removing the first protecting group to reveal one or more unprotected lysine type monomers;
(iii) converting the one or more unprotected lysine type monomers to one or more arginine type monomers; and
(iv) removing the second protecting group to obtain a peptoid, analogue or derivative thereof, comprising at least one arginine type monomer and at least one lysine type monomer.

Preferably, x is an integer between 0 and 9. More preferably, x is an integer between 0 and 5. Accordingly, x may be 0, 1, 2, 3, 4 or 5.

Accordingly, when x is 2, the arginine type monomer comprises an *N*-(3-guanidinopropyl) glycine (*N*Arg) monomer. Alternatively when x is 3 the arginine type monomer comprises *N*-(4-guanidinobutyl) glycine (*N*hArg) monomer, and when x is 1 the arginine type monomer comprises an *N*-(2-guanidinoethyl) glycine (*N*nArg) monomer.

Preferably, y is an integer between 0 and 9. More preferably, y is an integer between 0 and 5. Accordingly, y may be 0, 1, 2, 3, 4 or 5.

Accordingly, when y is 1, the lysine type monomer comprises an *N*-(2-aminoethyl) glycine (*N*ae) monomer. When y is 3, the lysine type monomer comprises an *N*-(4-aminobutyl) glycine (*N*Lys) monomer. When y is 5, the lysine type monomer comprises an *N*-(6-aminohexyl) glycine (*N*ah) monomer.

Advantageously, the method enables the synthesis of a peptoid, derivative or analogue thereof comprising at least one lysine type monomer and at least one arginine type monomer.

The term "derivative or analogue thereof" can mean that the amino acids residues of the peptoid are replaced by residues (whether natural amino acids, non-natural amino acids or amino acid mimics) with similar side chains or peptoid backbone properties. Additionally, the terminals of such peptoids may be protected by N- and C-terminal protecting groups with similar properties to acetyl or amide groups.

Derivatives and analogues of peptoids according to the invention may also include retropeptoid derivatives. A retropeptoid is expected to bind in the opposite direction in the ligand-binding groove, as compared to a peptide or peptoid-peptide hybrid containing one peptoid residue. As a result, the side chains of the peptoid residues are able point in the same direction as the side chains in the original peptide. Peptoid-peptide hybrids are also envisaged as derivatives or analogues described herein.

Preferably, the step of synthesising the linear precursor peptoid, analogue or derivative thereof comprises synthesising a linear precursor peptoid, analogue or derivative thereof on a substrate surface in a step-wise fashion.

The step of synthesising the linear precursor peptoid, analogue or derivative thereof may comprise:
- synthesising a first monomer on a substrate to obtain a linear precursor peptoid, analogue or derivative thereof comprising one monomer;
- subsequently adding at least one further monomer in a step wise fashion to obtain the linear precursor peptoid, analogue or derivative thereof containing the desired number of monomers.

It will be appreciated that the first and/or at least one further monomers maycomprise at least one lysine type monomer protected with a first protecting group and at least one lysine type monomer protected with a second protecting group. Additionally, the first and/or at least one further monomers may comprise at least one monomer comprising an aromatic residue and/or at least one monomer comprising an aliphatic residue.

The monomer comprising an aromatic residue may comprise an (S)-*N*-(1-phenylethyl) glycine (*N*spe) monomer, an (R)-*N*-(1-phenylethyl) glycine (*N*rpe) monomer, an *N-*(phenylmethyl) glycine (*N*phe) monomer, an *N*-(4-fluoro phenylmethyl) glycine (*N*pfb) monomer, an *N*-(3-fluoro phenylmethyl) glycine (*N*mfb) monomer, an (*S*)-*N*-1-(4-fluoro phenylethyl) glycine (*N*sfb) monomer, an (*R*)-*N*-1-(4-fluoro phenylethyl) glycine (*N*rfb) monomer, an *N*-(3,5 difluoro phenylmethyl) glycine (*N*dfb) monomer, an *N*-(4-chloro phenylmethyl) glycine (*N*pcb) monomer, an *N*-(4-methoxyphenylmethyl) glycine (*N*pmb) monomer, an *N*-(methylimidazole) glycine (*N*His) monomer, an *N-*(methylindole) glycine (*N*Trp) monomer, an *N*-(4-hydroxy phenylmethyl) glycine (*N*Tyr) monomer, an *N*-(4-pyridinylmethyl) glycine (*N*Pyr) monomer, an *(S)*-*N-*(1-naphthlethyl) glycine (*N*sna) monomer, an *(R)*-*N*-(1-naphthlethyl) glycine (*N*rna) monomer, an *N*-(furanylmethyl) glycine (*N*fur) monomer, an *N*-(thiofuranylmethyl) glycine (*N*tfur) monomer or an *N*-(diphenylmethyl) glycine (*N*dpa) monomer.

Preferably, the monomer comprises an *N*spe monomer or an *N*rpe monomer.

The monomer comprising an aliphatic residue may comprise an *N*-(pentyl) glycine (*N*amy) monomer, an *N*-(propyl) glycine (*N*NVa) monomer, an *N*-(isopentyl) glycine (*N*HLe) monomer, *N*-(isobutyl) glycine (*N*Leu) monomer, an *N*-(butyl) glycine (*N*but) monomer, an *N*-(2-carboxyethyl) glycine (*N*Glu) monomer, an *N*-(2,2,2-trifluoromethyl) glycine (*N*tfe) monomer, an *N*-(2,2,3,3,3-pentafluoropropyl) glycine (*N*pfp), an *N*-(2,2-difluoroethyl) glycine (*N*dfea) monomer, an *N*-(ethyl) glycine (*N*ea) monomer, an *N*-(2-thioethyl) glycine (*N*Cys) monomer, an (*S*)-*N*-(*sec*-butyl) glycine (*N*ssb) monomer, an (*R*)-*N*-(*sec*-butyl) glycine (*N*rsb) monomer, an (*S*)-*N*-(1-methylbutyl) glycine (*N*smb) monomer, an (*R*)-*N*-(1-methylbutyl) glycine (*N*rmb) monomer, an (*S*)-*N*-(1-cyclohexylethyl) glycine (*N*sch) monomer, (*R*)-*N*-(1-cyclohexylethyl) glycine (*N*rch) monomer, an *N*-(1-cyclohexylmethyl) glycine (*N*ch) monomer, an *N*-(ethynylmethyl) glycine (*N*em) monomer, an (*S*)-*N*-(1-ethynylethyl) glycine (Nsee) monomer, or an (*R*)-*N*-(1-ethynylethyl) glycine (*N*ree) monomer.

Accordingly, the precursor linear peptoid, analogue or derivative thereof may comprise one or more aliphatic and/or aromatic residues. The aliphatic and/or aromatic residues may be disposed at or towards the N-terminus, at or towards the C-terminus, or within the core of the peptoid, analogue or derivative thereof.

Advantageously, the method enables the synthesis of a peptoid, analogue or derivative thereof comprising lysine, arginine and aromatic residues.

The substrate preferably comprises a resin. The resin may comprise Rink amide resin, 2-chlorotrityl chloride resin or Wang resin, 4-(1',1'-dimethyl-1'-hydroxypropyl) phenoxyacetyl alanyl aminomethyl polystyrene (DHPP) resin or diphenyldiazomethane (PDDM) resin.

The step of synthesising a first monomer comprising on a substrate may comprise:
- contacting a substrate with haloacetic acid; and
- contacting the substrate with a desired amine sub-monomer to obtain a linear precursor peptoid, analogue or derivative thereof comprising one monomer.

The step of subsequently adding a further monomer may comprise:
- contacting the substrate with haloacetic acid; and
- contacting the substrate with a desired amine sub-monomer.

The step of subsequently adding a further monomer may be repeated until the linear precursor peptoid, analogue or derivative thereof contains the desired number of monomers.

The halo acetic acid preferably comprises bromoacetic acid.

Prior to the step of contacting the substrate with haloacetic acid, the method may comprise a step of contracting the substrate with a solvent. Preferably, the step of contacting the substrate with the solvent lasts for at least one hour. More preferably, the step of contacting the substrate with the solvent lasts for at least two, three, four or five hours. Most preferably, the step of contacting the substrate with the solvent lasts for at least six, seven, eight, nine or ten hours.

Preferably, the step of contacting the substrate with the solvent is undertaken at about room temperature.

Preferably, the solvent is dimethyl formamide (DMF), dichloromethane (DCM), dimethylacetamide (DMA) or *N*-methyl-2-pyrrolidone (NMP). Preferably, in embodiments where the substrate comprises Rink amide resin the solvent comprises dimethyl formamide (DMF). Preferably, in embodiments where the substrate comprises 2-chlorotrityl chloride resin the solvent comprises dichloromethane (DCM).

Preferably, the step of contacting the substrate with haloacetic acid is undertaken in the presence of a base. Preferably, the base if N,N'-diisopropylcarbodiimide (DIC) or N,N-Diisopropylethylamine (DIPEA).

Preferably, the step of contacting the substrate with haloacetic acid lasts for at least 5 minutes. More preferably, the step of contacting the substrate with haloacetic acid lasts for at least 10 or 15 minutes. Most preferably, the step of contacting the substrate with haloacetic acid lasts for at least 20 minutes.

Preferably, the step of contacting the substrate with haloacetic acid is undertaken at about room temperature.

Preferably, the molar ratio of the base to the substrate is at least 1:1. More preferably, the molar ratio of the base to the substrate is at least 2:1, 3:1, 4:1, 5:1, 6:1 or 7:1. Most preferably, the molar ratio of the base to the substrate is at least 8:1.

Preferably, the molar ratio of the haloacetic acid to the substrate is at least 1:1. More preferably, the molar ratio of the haloacetic acid to the substrate is at least 2:1, 3:1, 4:1, 5:1, 6:1 or 7:1. Most preferably, the molar ratio of the haloacetic acid to the substrate is at least 8:1.

Preferably, the step of contacting the substrate with the desired amine sub-monomer lasts for at least 5 minutes. More preferably, the step of contacting the substrate with the desired amine sub-monomer lasts for at least 10, 15, 20, 25 or 30 minutes. Most preferably, the step of contacting the substrate with the desired amine sub-monomer lasts for at least 35, 40, 45, 50, 55 or 60 minutes.

Preferably, the step of contacting the substrate with the desired amine sub-monomer is undertaken at about room temperature.

Preferably, the molar ratio of the desired amine sub-monomer to the substrate is at least 1:1. More preferably, the molar ratio of the desired amine sub-monomer to the substrate is at least 2:1, 3:1, 4:1, 5:1, 6:1 or 7:1. Most preferably, the molar ratio of the desired amine sub-monomer to the substrate is at least 8:1.

The desired amine sub-monomer may comprise a C₁₋₁₅ alkane substituted with an unprotected amino group on each terminal carbon, to obtain an unprotected lysine type monomer on the substrate.

Alternatively, the desired amine sub-monomer may comprise a C₁₋₁₅ alkane substituted with an unprotected amino group on a first terminal carbon and a protected amino group on a second terminal carbon, to obtain a protected lysine type monomer on the substrate. The protected amino group may be protected by the first protecting group or the second protecting group. Preferably, the protected amino group is protected by the Boc protecting group.

The C₁₋₁₅ straight chain alkane may comprise 1,2 diaminoethane, 1,3 diaminopropane, 1,4 diaminobutane, 1,5 diaminopentane, 1,6 diaminohexane, 1,7 diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane or 1,15-diaminopentadecane.

In embodiments where the monomer comprises an unprotected lysine type monomer the method may comprise an additional step carried out subsequent to contacting the substrate with the C₁₋₁₅ alkane, the subsequent step comprising contacting the unprotected lysine type monomer with a first further reagent.

Preferably, the step of contacting the unprotected lysine type monomer with the first further reagent lasts for at least 5 minutes. More preferably, the step of contacting the unprotected lysine type monomer with the first further reagent lasts for at least 10, 20, 30, 40, 50 or 60 minutes. Most preferably, the step of contacting the unprotected lysine type monomer with the first further reagent lasts for at least 70, 80, 90, 100, 110 or 120 minutes.

Preferably, the molar ratio of the first further reagent to the unprotected lysine type monomer is at least 1:1. More preferably, the first further reagent to the unprotected lysine type monomer is at least 2:1, 3:1, 4:1, 5:1, 6:1, 7:1, 8:1 or 9:1. Most preferably, the first further reagent to the unprotected lysine type monomer is at least 10:1.

Preferably, the step of contacting the unprotected lysine type monomer on the substrate with the first further reagent is undertaken at about room temperature.

Preferably, the first further reagent comprises 2-acetyldimedone (Dde-OH), di-tert-butyl dicarbonate ((Boc)₂O), 2,2,4,6,7-Pentamethyldihydrobenzofuran-5-sulfonyl chloride (Pbf-Cl), an alloc protecting group introducing reagent, 2,2,5,7,8-Pentamethyl-chromane-6-sulfonyl chloride (Pmc-Cl), [chloro-di(phenyl)methyl]benzene (Trt-Cl), t-Butyl Alcohol (tBuOH), tosyl chloride (Ts-Cl), tert-butyldimethylsilyl-chloride (TBDMS-Cl), Methoxytriphenylmethyl chloride (MMT-Cl), a Benzoyl (Z) protecting group introducing agent and a Benzyl (Bn) protecting group introducing agent. Most preferably, the first further reagent comprises 2-acetyldimedone (Dde-OH).

Alternatively, the desired amine sub-monomer may comprise a molecule comprise an amine and an aromatic or aliphatic group configured to obtain a monomer comprising an aromatic residue or aliphatic residue as defined above.

In embodiments where the first protecting group is Dde the step of removing the first protecting group to reveal one or more unprotected lysine type monomers may comprise contacting the peptoid, analogue or derivative thereof with hydrazine.

In embodiments where the second protecting group is Dde the step of removing the second protecting group to reveal one or more unprotected lysine type monomers may comprise contacting the peptoid, analogue or derivative thereof with hydrazine.

Preferably, the step of contacting the peptoid, analogue or derivative thereof with hydrazine lasts for at least 1 minutes. More preferably, the step of contacting the peptoid, analogue or derivative thereof with hydrazine lasts for at least 2 minutes. Most preferably, the step of contacting the peptoid, analogue or derivative thereof with hydrazine lasts for at least 3 minutes.

Preferably, the step of contacting the peptoid, analogue or derivative thereof with hydrazine is repeated at least once. More preferably, the step of contacting the peptoid, analogue or derivative thereof with hydrazine is repeated at least two or three times. Most preferably, the step of contacting the peptoid, analogue or derivative thereof with hydrazine is repeated at least four times.

Preferably, the step of contacting the peptoid, analogue or derivative thereof with hydrazine is undertaken at about room temperature.

In embodiments where the first protecting group is a tert-Butyloxycarbonyl (Boc) protecting group the step of removing the first protecting group to reveal one or more unprotected lysine type monomers may comprise contacting the peptoid, analogue or derivative thereof with a deprotecting solution comprising trifluoroacetic acid (TFA), water and/or triisopropylsilane (TIPS). Preferably, the step of removing the first protecting group to reveal one or more unprotected lysine type monomers comprises contacting the peptoid, analogue or derivative thereof with a deprotecting solution comprising trifluoroacetic acid (TFA), water and triisopropylsilane (TIPS).

In embodiments where the second protecting group is a tert-Butyloxycarbonyl (Boc) protecting group the step of removing the second protecting group to reveal one or more unprotected lysine type monomers may comprise contacting the peptoid, analogue or derivative thereof with a deprotecting solution comprising trifluoroacetic acid (TFA), water and/or triisopropylsilane (TIPS). Preferably, the step of removing the second protecting group to reveal one or more unprotected lysine type monomers comprises contacting the peptoid, analogue or derivative thereof with a deprotecting solution comprising trifluoroacetic acid (TFA), water and triisopropylsilane (TIPS).

Preferably, the deprotecting solution comprises at least 50% (v/v) trifluoroacetic acid (TFA). More preferably, the deprotecting solution comprises at least 60% (v/v), 70% (v/v), 80% (v/v) or 90% (v/v) trifluoroacetic acid (TFA). Most preferably, the deprotecting solution comprises about 95% (v/v) trifluoroacetic acid (TFA).

Preferably, the deprotecting solution comprises at least 0.5% (v/v) water. More preferably, the deprotecting solution comprises at least 1.0% (v/v), 1.5% (v/v) or 2.0% (v/v) water. Most preferably, the deprotecting solution comprises about 2.5% (v/v) water.

Preferably, the deprotecting solution comprises at least 0.5% (v/v) triisopropylsilane (TIPS). More preferably, the deprotecting solution comprises at least 1.0% (v/v), 1.5% (v/v) or 2.0% (v/v) triisopropylsilane (TIPS). Most preferably, the deprotecting solution comprises about 2.5% (v/v) triisopropylsilane (TIPS).

In a most preferred embodiment, the deprotecting solution comprises about 95% (v/v) trifluoroacetic acid (TFA), about 2.5% (v/v) water and about 2.5% (v/v) triisopropylsilane (TIPS).

Preferably, the step of contacting the peptoid, analogue or derivative thereof with a deprotecting solution lasts for at least 5 minutes. More preferably, the step of contacting the peptoid, analogue or derivative thereof with a deprotecting solution lasts for at least 10, 20, 30, 40 or 50 minutes. Most preferably, the step of contacting the peptoid, analogue or derivative thereof with a cleavage solution lasts for at least 60, 70, 80 or 90 minutes.

Preferably, the step of contacting the peptoid, analogue or derivative thereof with the deprotecting solution is undertaken at about room temperature.

The step of converting the one or more unprotected lysine type monomers to one or more arginine type monomers may comprise contacting the or each unprotected lysine type monomers with pyrazole-i-carboxamide. Preferably, the molar ratio of pyrazole-i-carboxamide to the or each unprotected lysine type monomer is at least 1:1. More preferably, the molar ratio of pyrazole-i-carboxamide to the or each unprotected lysine type monomer is at least 2:1, 3:1, 4:1 or 5:1. Most preferably, the molar ratio of pyrazole-i-carboxamide to the or each unprotected lysine type monomer is at least 6:1.

Preferably, the unprotected lysine type monomers are contacted with pyrazole-i-carboxamide in the presence of a base. Preferably, the base comprises diisopropylethylamine (DIPEA), triethylamine (TEA) or *N*-Methylmorpholine (NMM). Most preferably, the base comprises diisopropylethylamine (DIPEA). Preferably, the molar ratio of base to the or each unprotected lysine type monomer is at least 1:1. More preferably, the molar ratio of base to the or each unprotected lysine type monomer is at least 2:1, 3:1, 4:1 or 5:1. Most preferably, the molar ratio of base to the or each unprotected lysine type monomer is at least 6:1.

Preferably, the step of contacting the unprotected lysine type monomers with pyrazole-1-carboxamide lasts for at least 5 minutes. More preferably, the step of contacting the unprotected lysine type monomers with pyrazole-i-carboxamide lasts for at least 10, 20, 30, 40 or 50 minutes. Most preferably, the step of contacting the unprotected lysine type monomers with pyrazole-i-carboxamide lasts for at least 60, 70 or 80 minutes.

Preferably, the step of contacting the unprotected lysine type monomers with pyrazole-1-carboxamide is undertaken at about room temperature.

Preferably, the method comprises a step of cleaving the peptoid, derivative or analogue thereof from the substrate to obtain a cleaved precursor linear peptoid, analogue or derivative thereof.

The step of cleaving the substrate may comprise contacting the peptoid, derivative or analogue thereof with a cleavage solution. The cleavage solution may comprise trifluoroacetic acid (TFA), water, triisopropylsilane (TIPS), dichloromethane (DCM), acetic acid and/or hexafluoro-2-propanol (HFIP).

In one embodiment, the cleavage solution comprises trifluoroacetic acid (TFA).

The cleavage solution may comprise about 100% trifluoroacetic acid (TFA).

Alternatively, the cleavage solution may comprise trifluoroacetic acid (TFA) and dichloromethane (DCM).

Alternatively, the cleavage solution comprises trifluoroacetic acid (TFA), water and triisopropylsilane (TIPS). Preferably, in this embodiment, the substrate comprises Rink amide resin.

Preferably, the cleavage solution comprises at least 50% (v/v) trifluoroacetic acid (TFA). More preferably, the cleavage solution comprises at least 60% (v/v), 70% (v/v), 80% (v/v) or 90% (v/v) trifluoroacetic acid (TFA). Most preferably, the cleavage solution comprises about 95% (v/v) trifluoroacetic acid (TFA).

Preferably, the cleavage solution comprises at least 0.5% (v/v) water. More preferably, the cleavage solution comprises at least 1.0% (v/v), 1.5% (v/v) or 2.0% (v/v) water. Most preferably, the cleavage solution comprises about 2.5% (v/v) water.

Preferably, the cleavage solution comprises at least 0.5% (v/v) triisopropylsilane (TIPS). More preferably, the cleavage solution comprises at least 1.0% (v/v), 1.5% (v/v) or 2.0% (v/v) triisopropylsilane (TIPS). Most preferably, the cleavage solution comprises about 2.5% (v/v) triisopropylsilane (TIPS).

In a most preferred embodiment, the cleavage solution comprises about 95% (v/v) trifluoroacetic acid (TFA), about 2.5% (v/v) water and about 2.5% (v/v) triisopropylsilane (TIPS).

In an alternative embodiment, the cleavage solution comprises acetic acid.

In a further alternative embodiment, the cleavage solution comprises hexafluoro-2-propanol (HFIP). Preferably, in this embodiment the substrate comprises 2-chlorotrityl chloride resin.

Preferably, the step of contacting the peptoid, derivative or analogue thereof with a cleavage solution lasts for at least 5 minutes. More preferably, the step of contacting the peptoid, derivative or analogue thereof with a cleavage solution lasts for at least 10, 15, 20 or 25 minutes. Most preferably, the step of contacting the peptoid, derivative or analogue thereof with a cleavage solution lasts for at least 30 minutes.

Preferably, the step of contacting the peptoid, derivative or analogue thereof with a cleavage solution is undertaken at about room temperature.

Preferably, prior to the step of contacting the peptoid, derivative or analogue thereof with a cleavage solution the method comprises shrinking the substrate. Preferably, the step of shrinking the substrate comprises contacting the resin with ether.

In one embodiment, the cleaving step is carried out subsequent to step (i) and prior to step (ii). Accordingly, the precursor linear peptoid, derivative or analogue thereof may be cleaved from the substrate prior to the steps of removing either the first or second protecting groups. Preferably, the substrate comprises 2-chlorotrityl chloride resin. Preferably, the first and second protecting groups comprise Dde and a tert-Butyloxycarbonyl (Boc) protecting group.

Advantageously, when the substrate comprises 2-chlorotrityl chloride resin and the protecting groups comprise Dde and a tert-Butyloxycarbonyl (Boc) protecting group it is possible to cleave the precursor linear peptoid, derivative or analogue thereof from the substrate without removing either of the protecting groups.

The peptoid, analogue or derivative thereof, comprising at least one arginine type monomer and at least one lysine type monomer may comprise a linear peptoid, derivative or analogue thereof. Accordingly, step (ii) may be carried out on the cleaved precursor linear peptoid, analogue or derivative thereof.

Alternatively, the peptoid, analogue or derivative thereof, comprising at least one arginine type monomer and at least one lysine type monomer may comprise a cyclic peptoid, derivative or analogue thereof.

Accordingly, the method may comprise a cyclisation step comprising cyclising the precursor linear peptoid, derivative or analogue thereof to obtain a precursor cyclic peptoid. The cyclisation step may be carried out subsequent to the cleaving step. The cyclisation step may be carried out prior to step (ii). Accordingly, step (ii) may be carried out on the precursor cyclic peptoid, derivative or analogue thereof.

Advantageously, this will allow a user to synthesise a cyclic peptoid, derivative or analogue thereof comprising lysine and arginine type monomers.

The cyclisation step may comprise contacting the cleaved precursor linear peptoid, analogue or derivative thereof with a coupling reagent. The coupling reagent may comprise benzotriazol-i-yl-oxytripyrrolidinophosphonium hexafluorophosphate (PyBOP), N,N'-diisopropylcarbodiimide (DIC), N,N'-dicyclohexylcarbodiimide (DCC), ethyl (hydroxyimino)cyanoacetate (Oxyma) or O-(benzotriazol-1-yl)-N,N,N',N'-tetramethyluronium hexafluorophosphate (HBTU). Preferably, the molar ratio of coupling reagent to the cleaved precursor linear peptoid, analogue or derivative thereof is at least 1:1. More preferably, the molar ratio of coupling reagent to the cleaved precursor linear peptoid, analogue or derivative thereof is at least 2:1, 3:1, 4:1 or 5:1. Most preferably, the molar ratio of coupling reagent to the cleaved precursor linear peptoid, analogue or derivative thereof is at least 6:1.

The step of contacting the cleaved precursor linear peptoid, analogue or derivative thereof with coupling reagent may be conducted in the presence of a base. The base may comprise N,N-Diisopropylethylamine (DIPEA), triethylamine (TEA) or N-Methylmorpholine (NMM). Preferably, the base comprises N,N-Diisopropylethylamine (DIPEA). Preferably, the molar ratio of the base to the cleaved precursor linear peptoid, analogue or derivative thereof is at least 1:1. More preferably, the molar ratio of the base to the cleaved precursor linear peptoid, analogue or derivative thereof is at least 2:1, 3:1, 4:1 or 5:1. Most preferably, the molar ratio of the base to the cleaved precursor linear peptoid, analogue or derivative thereof is at least 6:1.

Preferably, the step of contacting the cleaved precursor linear peptoid, analogue or derivative thereof with coupling reagent lasts for at least 5 minutes. More preferably, the step of contacting the cleaved precursor linear peptoid, analogue or derivative thereof with coupling reagent lasts for at least 10, 20, 30, 40 or 50 minutes. Most preferably, the step of contacting the cleaved precursor linear peptoid, analogue or derivative thereof with coupling reagent lasts for at least 60 minutes.

Preferably, the step of contacting the cleaved precursor linear peptoid, analogue or derivative thereof with coupling reagent is undertaken at about room temperature.

In an alternative embodiment, the cleaving step may be carried out subsequent to step (iii). Accordingly, the cleaving step may be carried out prior to the step of removing the second protecting group. Alternatively, the cleaving step may be carried out subsequent to the step of removing the second protecting group. However, in a preferred embodiment the cleaving step is carried out simultaneously to the step of removing the second protecting group. In this embodiment the substrate may comprise Rink amide resin and the second protecting group may comprise a tert-Butyloxycarbonyl (Boc) protecting group.

Advantageously, in embodiments where the substrate comprises Rink amide resin and the second protecting group comprises a tert-Butyloxycarbonyl (Boc) protecting group the conditions used to remove the second protecting group also cleave the peptoid, derivative or analogue thereof from the substrate. Advantageously, by cleaving the peptoid, derivative or analogue thereof from the substrate at the same time as removing the second protecting group, a linear peptoid, derivative or analogue thereof is obtained.

The inventors believe that the peptoids which may be obtained using the above method are novel *per se.*

Hence, in accordance with a second aspect, there is provided a peptoid, analogue or derivative thereof, wherein the peptoid, analogue or derivative thereof comprises:
- a linear peptoid, analogue or derivative thereof which has the structure (*N*Lys-*N*spe-*N*spe)₂(*N*hArg-*N*spe-*N*spe)₂; (*N*hArg-*N*spe-*N*spe)₂(*N*Lys-*N*spe-*N*spe)₂; (*N*Lys-*N*spe-*N*spe)(*N*hArg-*N*spe-*N*spe)(*N*Lys-*N*spe-*N*spe)₂; [(*N*hArg-*N*spe-*N*spe)(*N*Lys-*N*spe-*N*spe)]2; or [(*N*nArg*N*spe*N*spe)(*N*ae*N*spe*N*spe)]₂; or
- a cyclic peptoid, analogue or derivative thereof which has the structure (*N*Lys-*N*phe-*N*hArg-*N*phe-*N*Lys-*N*phe).

Preferably, the peptoid, analogue or derivative thereof is obtained using the method of the first aspect.

The inventors believe that peptoids made in accordance with the present invention may be used as medicaments.

Therefore, in accordance with a third aspect there is provided a peptoid, analogue or derivative thereof according to the second aspect, for use as a medicament.

In accordance with a fourth aspect, there is provided a peptoid, analogue or derivative thereof according to the second aspect, for use in treating, ameliorating or preventing a microbial infection.

Examples 3 to 5 summarise the surprising antibacterial activity of the peptoids synthesised using methods of the invention.

Accordingly, the microbial infection may comprise a bacterial infection, a fungal infection or a parasitic infection.

In one embodiment, the microbial infection comprises a bacterial infection. The bacterial infection may comprise a gram positive bacterial infection or a gram negative bacterial infection. The bacterium may be from the *Escherichia* genus, preferably E. *coli.* The bacterium may be from the *Pseudomonas* genus, preferably *P. aeruginosa.* The bacterium may be from the *Staphylococcus* genus, preferably *S. aureus.* The bacterium may be from the *Serratia* genus, preferably *S. marcesens.*

In one embodiment, the microbial infection comprises a fungal infection. The fungus may be from the *Candida* genus, preferably C. *albicans.*

In one embodiment, the microbial infection comprises a parasitic infection. The parasite may be a protozoan parasite.

The parasite may be from the *Leishmania* genus, preferably *L. mexicana* or *L. donovani.* Accordingly, the peptoid, analogue or derivative thereof may be for use in treating, ameliorating or preventing leishmaniasis, preferably cutaneous leishmaniasis or visceral leishmaniasis.

The parasite may be from the *Trypanosoma* genus. The parasite may be *T. brucei,* preferably *T. brucei rhodesiense.* Accordingly, the peptoid, analogue or derivative thereof may be for use in treating, ameliorating or preventing African trypanosomiasis. It will be appreciated that African trypanosomiasis is known as African sleeping sickness in humans. Accordingly, the peptoid, analogue or derivative thereof may be for use in treating, ameliorating or preventing African sleeping sickness. Alternatively, the parasite may be *T. cruzi.* Accordingly, the peptoid, analogue or derivative thereof may be for use in treating, ameliorating or preventing Chagas disease.

The parasite may be from the *Plasmodium* genus, preferably *P. falciparum.* Accordingly, the peptoid, analogue or derivative thereof may be for use in treating, ameliorating or preventing malaria.

It will be appreciated that peptoids, derivatives or analogues thereof described herein may be used in a medicament which may be used in a monotherapy (i.e. use of the compound alone), for treating, ameliorating, or preventing a microbial infection. Alternatively, the peptoids, derivatives or analogues thereof described herein may be used as an adjunct to, or in combination with, known therapies for treating, ameliorating, or preventing a microbial infection.

The peptoids, derivatives or analogues thereof described herein may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

Medicaments comprising the peptoids, derivatives or analogues thereof described herein may be used in a number of ways. For instance, oral administration may be required, in which case the compound may be contained within a composition that may, for example, be ingested orally in the form of a tablet, capsule or liquid. Compositions comprising the compounds of the invention may be administered by inhalation (e.g. intranasally). Compositions may also be formulated for topical use. For instance, creams or ointments may be applied to the skin.

Compounds according to the invention may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with compounds used according to the invention is required and which would normally require frequent administration (e.g. at least daily injection).

In a preferred embodiment, peptoid, derivative or analogue thereof may be administered to a subject by injection into the blood stream or directly into a site requiring treatment. Injections may be intravenous (bolus or infusion) or subcutaneous (bolus or infusion), or intradermal (bolus or infusion).

It will be appreciated that the amount of the peptoid, derivative or analogue thereof that is required is determined by its biological activity and bioavailability, which in turn depends on the mode of administration, the physiochemical properties of the compound, and whether it is being used as a monotherapy, or in a combined therapy. The frequency of administration will also be influenced by the half-life of the compound within the subject being treated. Optimal dosages to be administered may be determined by those skilled in the art, and will vary with the particular compound in use, the strength of the pharmaceutical composition, the mode of administration, and the advancement of the microbial infection. Additional factors depending on the particular subject being treated will result in a need to adjust dosages, including subject age, weight, gender, diet, and time of administration.

Generally, a daily dose of between 0.01µg/kg of body weight and 500mg/kg of body weight of the peptoid, derivative or analogue thereof may be used for treating, ameliorating, or preventing a microbial infection depending upon which peptoid is used. More preferably, the daily dose is between 0.01mg/kg of body weight and 400mg/kg of body weight, more preferably between 0.1mg/kg and 200mg/kg body weight, and most preferably between approximately 1mg/kg and 100mg/kg body weight.

The peptoid, derivative or analogue thereof may be administered before, during or after onset of microbial infection to be treated. Daily doses may be given as a single administration (e.g. a single daily injection). Alternatively, the microbial infection may require administration twice or more times during a day. As an example, a compound according to the second aspect may be administered as two (or more depending upon the severity of the microbial infection being treated) daily doses of between 25mg and 7000 mg (i.e. assuming a body weight of 70 kg). A patient receiving treatment may take a first dose upon waking and then a second dose in the evening (if on a two dose regime) or at 3- or 4-hourly intervals thereafter. Alternatively, a slow release device may be used to provide optimal doses of the compounds according to the invention to a patient without the need to administer repeated doses.

Known procedures, such as those conventionally employed by the pharmaceutical industry (e.g. *in vivo* experimentation, clinical trials, etc.), may be used to form specific formulations comprising the peptoid, derivative or analogue thereof and precise therapeutic regimes (such as daily doses of the compounds and the frequency of administration). The inventors believe that they are the first to describe a pharmaceutical composition for treating a microbial infection, based on the use of the peptoid, derivative or analogue thereof according to the invention.

Hence, in a fifth aspect of the invention, there is provided a pharmaceutical composition comprising a peptoid, analogue or derivative thereof according to the second aspect, and a pharmaceutically acceptable vehicle.

The pharmaceutical composition can be used in the therapeutic amelioration, prevention or treatment in a subject of a microbial infection. Thus, the composition is preferably an antimicrobial pharmaceutical composition. Most preferably, the composition is an antibacterial composition.

The invention also provides, in a sixth aspect, a process for making the composition according to the fifth aspect, the process comprising contacting a therapeutically effective amount of a peptoid, analogue or derivative thereof according to the second aspect and a pharmaceutically acceptable vehicle.

A "subject" maybe a vertebrate, mammal, or domestic animal. Hence, compounds, compositions and medicaments according to the invention may be used to treat any mammal, for example livestock (e.g. a horse), pets, or may be used in other veterinary applications. Most preferably, however, the subject is a human being.

A "therapeutically effective amount" of a peptoid, analogue or derivative thereof according to the second aspect is any amount which, when administered to a subject, is the amount of drug that is needed to treat the target disease, or produce the desired effect, i.e. prevent or reduce the microbial infection.

For example, the therapeutically effective amount of peptoid, analogue or derivative thereof used may be from about 0.01 mg to about 800 mg, and preferably from about 0.01 mg to about 500 mg. It is preferred that the amount of compound is an amount from about 0.1 mg to about 250 mg, and most preferably from about 0.1 mg to about 20 mg.

A "pharmaceutically acceptable vehicle" as referred to herein, is any known compound or combination of known compounds that are known to those skilled in the art to be useful in formulating pharmaceutical compositions.

In one embodiment, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents (i.e. the compounds described herein) according to the invention. In tablets, the active compound may be mixed with a vehicle having the necessary compression properties in suitable proportions and compacted in the shape and size desired. The powders and tablets preferably contain up to 99% of the active compound. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The compounds according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions, which are sterile solutions or suspensions, can be utilized by, for example, intramuscular, intrathecal, epidural, intraperitoneal, intravenous and particularly subcutaneous injection. The compound may be prepared as a sterile solid composition that may be dissolved or suspended at the time of administration using sterile water, saline, or other appropriate sterile injectable medium.

The compound and compositions of the invention may be administered in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The compounds used according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

All features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-
**Figure 1** shows the comparative structures of an exemplary peptide and a corresponding exemplary peptoid;
**Figure 2** shows a prior art reaction sequence for synthesising guanidinylate peptoids;
**Figure 3A** shows a Dde-protected *N*Lys residue used in a method in accordance with an embodiment of the invention;
**Figure 3B** shows a Boc-protected *N*Lys residue used in a method in accordance with an embodiment of the invention;
**Figure 4** is a reaction scheme according to an embodiment of the invention showing how the Dde-protected *N*Lys residue of Figure 3A can be synthesised and incorporated into an extended peptoid chain;
**Figure 5** is a reaction scheme according to another embodiment of the invention showing how a linear mixed peptoid can be synthesised;
**Figure 6** is a reaction scheme according to another embodiment of the invention showing how a cyclic mixed peptoid can by synthesised;
**Figure** 7 shows the structure of a first embodiment of a peptoid referred to herein as "peptoid 1";
**Figure 8** shows the structure of a second embodiment of a peptoid referred to herein as "peptoid 2";
**Figure 9** shows the structure of a third embodiment of a peptoid referred to herein as "peptoid 3";
**Figure 10** shows the structure of a fourth embodiment of a peptoid referred to herein as "peptoid 4";
**Figure 11** shows the structure of a fifth embodiment of a peptoid referred to herein as "peptoid 5";
**Figure 12** is a generic structure for a peptoid in accordance with the present invention;
**Figure 13** shows monomers comprising aromatic residues which may comprise part of a peptoid of the present invention;
**Figure 14** shows monomers comprising aliphatic residues which may comprise part of a peptoid of the present invention;
**Figure 15A** shows a Dde-protected Nae residue used in a method in accordance with an embodiment of the invention;
**Figure 15B** shows a Boc-protected Nae residue used in a method in accordance with an embodiment of the invention;
**Figure 16** shows the structure of a sixth embodiment of a peptoid referred to herein as "peptoid 6";
**Figure 17A** is a graph showing the activity of peptoids 1-4 against C. *albicans*;
**Figure 17B** is a graph showing the activity of peptoids 1-4 against *S. aureus*; and
**Figure 17C** is a graph showing the activity of peptoids 1-4 against *E. coli.*

### Example 1 - Synthesis of linear mixed peptoids

The inventors have developed a novel method using orthogonal protecting groups to protect lysine type residues, such as *N*Lys residues and *N*ae residues, to synthesise linear mixed peptoids containing lysine type resides, arginine type residues and aromatic side chains.

### Materials and methods

### Procedure 1 - On resin peptoid synthesis

Fmoc-protected Rink Amide resin (normally 100-300 mg, 0.1-0.3 mmol, typical loading between 0.6-0.8 mmol g-1) was swollen in DMF (at least 1 hour, overnight preferred, at room temperature) in a 20 mL polypropylene syringe fitted with two polyethylene frits (Crawford Scientific). The resin was deprotected with piperidine (20% in DMF v/v, 2 × 20 min) and washed with DMF (3 × 2mL). The resin was treated with bromoacetic acid (1 ml, 0.6M in DMF) and DIC (0.18 ml, 50% v/v in DMF) for 20 minutes at room temperature at 400 rpm on a shaker platform. The resin was washed with DMF (3 × 2 mL), before the desired amine sub-monomer was added (1 ml, 0.8-2M in DMF) and allowed to react for 60 minutes at room temperature on the shaker. The resin was again washed with DMF (3 × 2 mL).

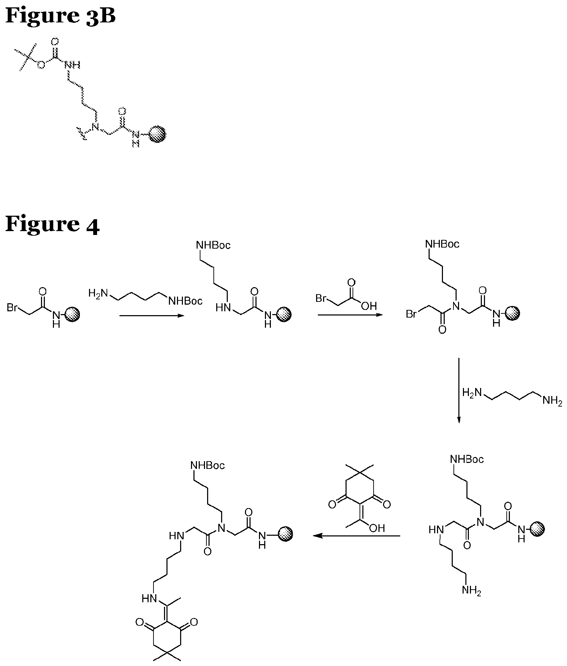

For instance, when the desired monomer was a Boc protected *N*Lys monomer the desired amine sub-monomer was *N*-Boc 1,4 diaminobutane, as shown in step 1 of Figure 4. Alternatively, when the desired monomer unit was a Boc protected *N*ae monomer unit the desired amine sub-monomer was *N*-Boc 1,2 diaminoethane.

When the desired monomer was a Dde protected *N*Lys monomer the desired amine sub-monomer was 1,4 diaminobutane, as shown in step 3 of Figure 4. Alternatively, when the desired monomer unit was a Dde protected *N*ae monomer unit the desired amine sub-monomer was 1,2 diaminoethane. In both these cases procedure 2 would then be conducted to add the Dde protecting group before procedure 1 was repeated.

When the desired monomer was *N*spe the amine sub-monomer was (*S*)-(-)-α-methylbenzylamine.

Procedure 1 was repeated until the desired peptoid sequence had been obtained. Once the desired full length orthogonally protected peptoid had been obtained procedures 3 to 5 were undertaken sequentially.

### Procedure 2 - Dde protection of NLys submonomer

Dde-OH (10 eq. wrt resin, 1M in DMF) was added to the resin and placed on the shaker at RT for 60 minutes, then the resin was washed well with DMF (3 × 2mL).

As explained above, subsequent peptoid couplings were then made by repeating procedure 1 until the desired full length peptoid sequence was obtained.

### Procedure 3 - Guanidinylation of the free amines

On resin deprotection of the Dde group was undertaken using 2% hydrazine in DMF (4 × 4ml × 3 mins) and then the resin washed with DMF (3 × 2 mL). Guanidinylation of the free amines were undertaken using pyrazole-i-carboxamide (6 eq. per free amine, in the minimum amount of DMF) and DIPEA (6 eq. per free amine) on the shaker at 400 rpm, RT for 90 minutes.

### Procedure 4 - Cleavage of peptoid from substrate

The resin was washed with DMF (3 × 2 mL) before cleavage. Final cleavage from resin was achieved using 95:2.5:2.5 TFA : H2O : TIPS (4ml) for 1.5 hours and the resin removed by filtration. The cleavage cocktail was removed in vacuuo, the crude product precipitated in diethyl ether (45 mL) and the precipitate retrieved by centrifuge for 15 min at 5,000 rpm. The ether phase was decanted, the crude product dissolved in a mixture of acidified H2O (0.1% TFA) and MeCN and lyophilised.

### Procedure 5 - Purification of crude peptoids

Crude peptoids were dissolved into ~1.5 mL (95% H2O, 5% MeCN, 0.1% TFA) and purified by preparative RP-HPLC using a Perkin Elmer 200 Series LC pump with a Perkin-Elmer 785A UV-vis detector (λ = 250nm) on a SB Analytical column (ODS-H Optimal), 250 × 10mm, 5 µm; flow rate = 2 mL min-1; linear gradient elution 0-50% solvent B over 60 minutes, then 50-100% B over 15 minutes (solvent A = 0.1% TFA in 95% H2O, 5% MeCN, solvent B = 0.1% TFA in 5% H20, 95% MeCN). Relevant fractions were collected, lyophilized and analysed by LC-MS and analytical RP-HPLC.

All peptoids were obtained with >95% purity.

### Results

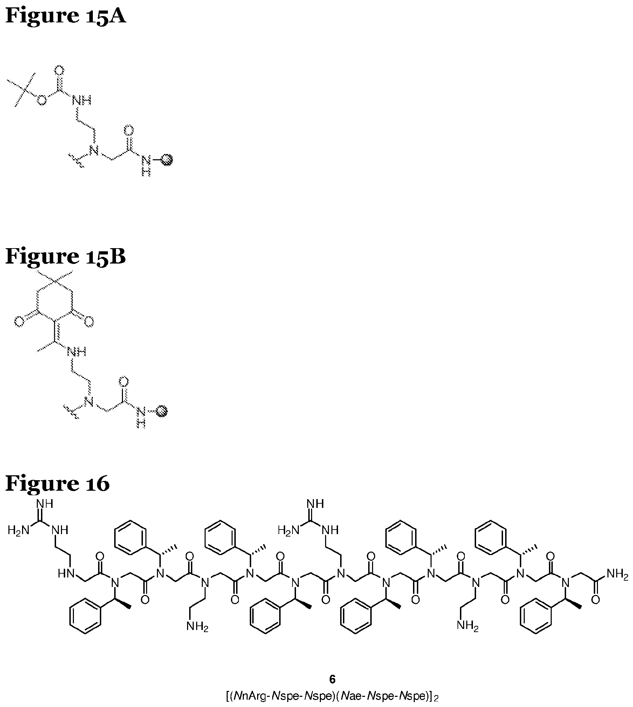

*N*Lys residues protected with Boc and Dde protecting groups are shown in Figures 3A and 3B respectively, and *N*ae residues protected with Boc and Dde protecting groups are shown in Figures 15A and 15B respectively.

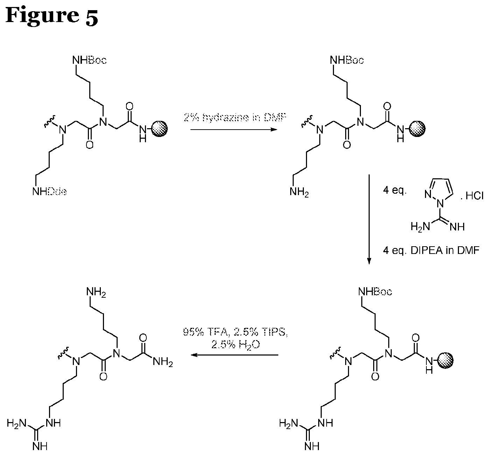

For the synthesis of linear peptoids the procedures 1 and 2, given above, were used in combination to assemble the orthogonally protected specific peptoid sequence on a resin, as shown in Figure 4. Using procedure 3, Dde was then easily removed using 2% hydrazine in DMF which allowed these residues to be selectively deprotected, while leaving Boc protection on other lysine type chains intact, as shown in step 1 of Figure 5. The deprotected lysine type residues can then selectively undergo guanidinylation to introduce arginine type residues, as shown in step two of Figure 5. Using procedure 4, the peptoids can then be cleaved from the resin, and the Boc protection group can be removed in one final step to obtain a mixed peptoid containing both lysine type residues and arginine type residues, as shown in step 3 of Figure 5. Finally, using procedure 5, the peptoids can be purified.

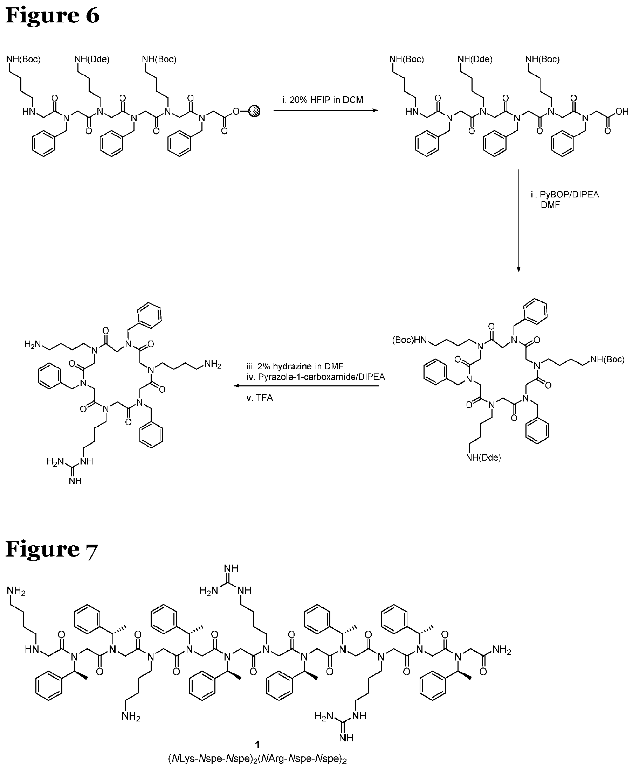

Five different embodiments of linear peptoids, referred to as peptoids 1 to 4 and 6, were prepared using the above methodology. The structures of the peptoids are shown in Figures 7 to 10 and 16, and are:
**Peptoid 1** has the structure (*N*Lys-*N*spe-*N*spe)₂(NhArg-*N*spe-*N*spe)₂ - [SEQ ID No:1];
**Peptoid 2** has the structure (NhArg-*N*spe-*N*spe)₂(*N*Lys-*N*spe-*N*spe)₂ - [SEQ ID No:2];
**Peptoid 3** has the structure (*N*Lys-*N*spe-*N*spe)(NhArg-*N*spe-*N*spe)(*N*Lys-*N*spe-*N*spe)₂ - [SEQ ID No:3];
**Peptoid 4** has the structure [(NhArg-*N*spe-*N*spe)(*N*Lys-*N*spe-*N*spe)]₂ - [SEQ ID No:4];
**Peptoid 6** has the structure [(*N*nArg*N*spe*N*spe)(*N*ae*N*spe*N*spe)]₂ - [SEQ ID No:6].

All of the peptoids are 12 residue linear peptoids. Peptoids 1, 2 and 4 each contain two lysine type monomers (*N*Lys), two arginine type monomers (NhArg) and eight aromatic residues (*N*spe i.e. (*S*)-*N*-1-phenylethyl), as shown in Figures 7, 8 and 10.

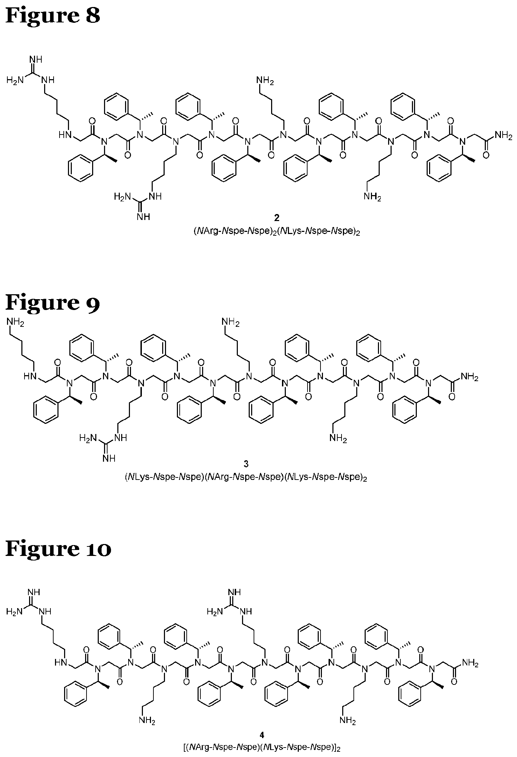

Peptoid 3 contains three lysine type monomers (*N*Lys), one arginine type monomer (*N*hArg) and eight aromatic residues (*N*spe i.e. (S)-*N*-1-phenylethyl), as shown in Figure 9.

Peptoid 6 contains two lysine type monomers (*N*ae) and two arginine type monomers (*N*nArg).

### Conclusion

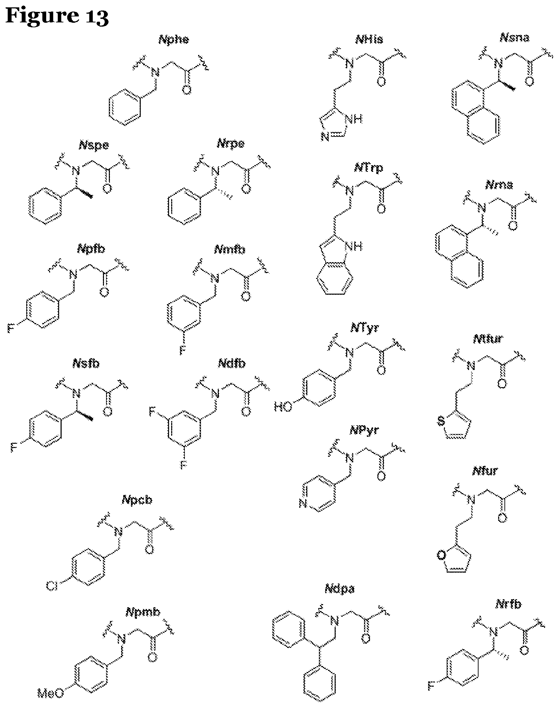

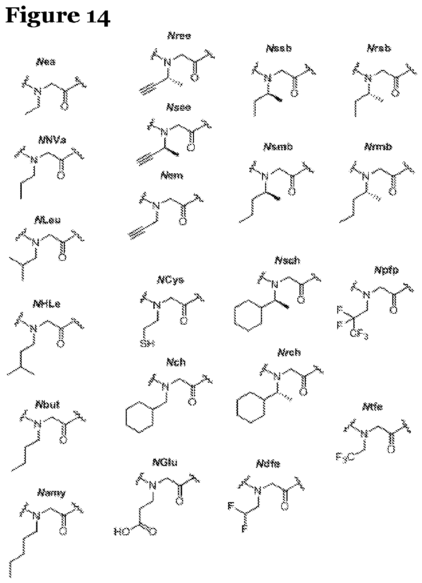

The inventors have found that, by using orthogonal protecting groups to protect lysine type residues, it is possible to synthesise linear peptoids containing lysine type residues, arginine type residues and aromatic side chains. This has not been possible previously. While *N*spe is used as an additional monomer in this example it will be appreciated that further monomers could be used. Examples of appropriate monomers comprising aromatic residues are shown in Figure 13 and examples of appropriate monomers comprising aliphatic residues are shown in Figure 14.

The orthogonal protecting groups used in this example are Boc and Dde. However, it will be appreciated that alternative protecting groups could be used.

The method devised by the inventors is a versatile method allowing the Dde-protecting group to be added and selectively deprotected in a variety of positions in the peptoid sequence, both near C and N-terminal positions and also within close proximity to each other.

### Example 2 - Synthesis of cyclic mixed peptoids

The inventors have developed a novel method using orthogonal protecting groups to synthesise cyclic mixed peptoids comprising lysine and arginine side chains.

### Materials and Methods

### Procedure 1 - On resin peptoid synthesis

2-chlorotrityl chloride resin (0.1 mmol, typical loading 1.22 mmol g⁻¹) was swollen in dry DCM (45 mins, at room temperature) in a 20 mL polypropylene syringe fitted with two polyethylene frits. The resin was washed with dry DCM (3 × 2 mL) and loaded with bromoacetic acid (1 ml, 0.6 M in DMF) and neat DIPEA (16 eq. with respect to the resin) for 30 minutes at RT on a shaker at 400 rpm. The resin was washed with DMF (3 × 2 mL), before the desired amine sub-monomer was added (1 ml, 1.5 M in DMF) and allowed to react for 60 minutes at RT on the shaker.

For instance, when the desired monomer was a Boc protected *N*Lys monomer the desired amine sub-monomer was *N*-Boc 1,4 diaminobutane, as shown in step 1 of Figure 4.

When the desired monomer was a Dde protected *N*Lys monomer the desired amine sub-monomer was 1,4 diaminobutane, as shown in step 3 of Figure 4. In this case procedure 2 would then be conducted to add the Dde protecting group before procedure 1 was repeated.

When the desired monomer was *N*phe the amine was benzylamine.

Procedure 1 was repeated until the desired peptoid sequence had been obtained. Once the desired full length orthogonally protected peptoid had been obtained procedures 3 to 7 were undertaken sequentially.

### Procedure 2 - Dde protection of NLys submonomer

Dde-OH (10 eq. wrt resin, 1M in DMF) was added to the resin and placed on the shaker at RT for 60 minutes, then the resin washed well with DMF (3 × 2mL).

As explained above, subsequent peptoid couplings were then made by repeating procedure 1 until the desired full length peptoid sequence was obtained.

### Procedure 3 - Cleavage of peptoid from substrate

Final cleavage from resin was achieved using HFIP (4 mL, 20% v/v in DCM) for 30 minutes. The resin was removed by filtration and the cleavage cocktail sparged off using a fine stream of N₂. The crude product was precipitated in diethyl ether (15 mL) and the precipitate retrieved by centrifuge for 15 min at 5,000 rpm. The ether phase was decanted, the crude, protected product dissolved in a mixture of acidified H₂O (0.1% TFA) and MeCN and lyophilised.

### Procedure 4 - Cyclisation of peptoid

The crude peptoid was cyclised in solution without further purification. The linear peptoid (100 µmol) was dissolved in dry DMF (10 mL) and added dropwise to a solution of PyBOP and DIPEA (both 6 eq. with respect to the crude linear peptoid, in 10 mL DMF) over 8 hours. The reaction was allowed to proceed for a further 60 minutes at room temperature following the last addition. The DMF solvent was removed *in vaccuo* and the crude peptoids were extracted using DCM (2 × 20 mL). The organic phases were combined, washed with water and dried over MgSO₄ before filtration and solvent removal *in vacuuo.* The resulting residue was dissolved in 50% MeCN in H₂O and lyophilised.

The protected peptoids were then dissolved in 50% MeCN in H₂O and purified by preparative RP-HPLC; flow rate = 2 mL min⁻¹; injection made at 50% B and a linear gradient elution 50-100% solvent B over 60 minutes (solvent A = 0.1% TFA in 95% H₂O, 5% MeCN, solvent B = 0.1% TFA in 5% H₂O, 95% MeCN). Relevant fractions were collected, lyophilized and analyzed by LC-MS.

### Procedure 5 - Guanidinylation of the free amines

At this stage, Dde-groups were removed using 2% hydrazine in DMF (4 × 4ml × 3 mins) and then the resin washed with DMF (3 × 2 mL). Guanidinylation of the free amines were undertaken using pyrazole-i-carboxamide (6 eq. per free amine, in the minimum amount of DMF) and DIPEA (6 eq. per free amine) on the shaker at 400 rpm, RT for 90 minutes.

### Procedure 6 - Removal of Boc protecting groups

The cyclic peptoids were then Boc-deprotected using 95:2.5:2.5 TFA: H₂O : TIPS (4ml) for 1.5 hours. The cleavage cocktail was removed *in vacuuo,* the crude product precipitated in diethyl ether (45 mL) and the precipitate retrieved by centrifuge for 15 min at 5,000 rpm. The ether phase was decanted, the crude product dissolved in a mixture of acidified H₂O (0.1% TFA) and MeCN and lyophilised.

### Procedure 7 - Purification of crude peptoids

The peptoids were dissolved in ~1.5 mL (95% H2O, 5% MeCN, 0.1% TFA) and purified by preparative RP-HPLC flow rate = 2 mL min⁻¹; linear gradient elution 0-50% solvent B over 60 minutes, then 50-100% B over 15 minutes. Relevant fractions were collected, lyophilized and analyzed by LC-MS and analytical RP-HPLC.

All peptoids were obtained with >95% purity.

### Results

Linear precursors were synthesised on 2-chlorotrityl chloride resin using procedures 1 and 2, given above. Again, this reaction sequence will be as shown in Figure 4. Using procedure 3, the linear precursors were cleaved from the resin without removing either the Boc or the Dde protecting groups, as shown in step 1 of Figure 6. Using procedure 4, a head-to-tail, solution phase cyclisation was then undertaken, as shown in step 2 of Figure 6. Following cyclisation, the crude cyclic species was purified via RP-HPLC and then, using procedure 5, the Dde groups could be selectively deprotected and a guanidinylation reaction carried out in solution. Finally, the Boc protecting groups could also be removed, using procedure 6. These two procedures are shown as the final step in Figure 6. Finally, using procedure 7 RP-HPLC purification was carried out to obtain peptoids with > 95% purity.

One cyclic peptoid, referred to as peptoid 5, was prepared using the above methodology. The structure of the peptoid is shown in Figure 11, and is cyclic (*N*Lys-*N*phe-*N*hArg-*N*phe-*N*Lys-*N*phe) - [SEQ ID No:5].

Peptoid 5 is a six residue cyclic peptoid containing two lysine type monomers (*N*Lys), one arginine type monomer (*N*hArg), and three aromatic residues (*N*phe ).

### Conclusion

The synthesis of peptoid 5 shows that by using orthogonal protecting groups, it is possible to synthesise cyclic peptoids comprising lysine and arginine side chains. The inventors believe that this is the first example of a cyclic peptoid that contains an Arg type monomer in combination with a Lys type monomer.

While *N*phe is used as an additional monomer in this example it will be appreciated that, as with Example 1, further monomers could be used.

As with example 1, the orthogonal protecting groups used in this example are also Boc and Dde. However, it will be appreciated that alternative protecting groups could be used.

Despite the bulky nature of the Dde group, the cyclisation reaction still occurred efficiently at room temperature and complete cyclisation was possible.

### Example 3 - Biological data: planktonic bacteria

The inventors have demonstrated that the mixed peptoids prepared as described above exhibit surprising antibacterial properties against planktonic bacteria.

### Materials and methods

### Bacterial Strains

Species used in MIC assays included gram-negative *Escherichia coli* K12 W3110, *Pseudomonas aeruginosa* laboratory strain PAO2 and *Serratia marcescens* laboratory strain and gram-positive *Staphylococcus aureus* NCTC 6571 and *Micrococcus luteus* laboratory strain.

### Overnight Culture Preparation

Bacterial cultures were prepared by streaking the bacterial strains on to agar plates with an inoculation loop and incubating overnight at 37°C. A single colony was then selected and placed in 5 mL of Iso-Sensitest broth using an inoculation loop and incubated at 37 °C with shaking overnight.

### MIC Determination

MIC values were attained according to the protocol described by J. M. Andrews *et al.* [J. M. Andrews, *J. Antimicrob. Chemother.,* 2001, **48**, 5-16] and were conducted in 96-well microtitre plates in triplicate. 10-50 µL of each overnight culture was inoculated into 1.2 mL of Iso-Sensitest broth and grown at 37°C with shaking. An inoculum density of ~10⁴ cfu/spot was determined by comparison with 0.5 MacFarland standard (240 µM BaCl₂ in 0.18 M H₂SO_{4 aq.}) and was found to relate to an A₆₅₀ₙₘ of 0.07 after calibration with regular Iso-Sensitest broth. The inoculum was diluted ten-fold with Iso-Sensitest broth before use (to ~10³ cfu/spot). Peptide solutions were initially dissolved in DMSO (5 mg mL⁻¹) and then diluted further with Iso-Sensitest broth to achieve a concentration range of 4 mgL⁻¹ to 512 mgL⁻¹ using 2-fold serial dilutions. Samples were vortexed between dilutions where necessary to aid dissolution. 50 µL of inoculum and 50 µL of peptide solution were added to each test well to achieve a final concentration range of 2 mgL⁻¹ to 256 mgL⁻¹. Separate dilutions of ampicillin and DMSO were made up in a similar manner to act as a positive antibacterial control and a +DMSO control, respectively. 50 µL of inoculum and 50 µL of Iso-Sensitest broth were used as a positive control and 100 µL of inoculum was used as a negative control. Positive and negatives controls were conducted multiple times in parallel per plate. MIC was defined as the lowest concentration which completely inhibited bacterial growth after incubation at 37°C for 16 h with shaking. IC₅₀ was defined as the concentration of antibiotic which achieved a 50% inhibition of bacterial growth after incubation at 37°C for 16 h with shaking. Quantitative data were attained as A₆₅₀ₙₘ values using a BioTek^{®} Synergy^{™} H4 Hybrid Multi-Mode Microplate Reader.

### Results

The anti-bacterial activity of peptoids 1 to 4 are shown in Table 1.

**Table 1: Anti-bacterial properties of peptoids 1 to 4**

| **Peptoid** | **MIC (µM** / **mgL⁻¹)** | | | |
|---|---|---|---|---|
| | *E.coli* | *P.aeruginosa* | *S.aureus* | *S*. *marcescens* |
| 1 | 17/32 | 34/64 | 17/32 | 134/256 |
| 2 | 17/32 | 17/32 | 17/32 | 134/256 |
| 3 | 17/32 | 17/32 | 17/32 | 134/256 |
| 4 | 17/32 | 67/128 | 17/32 | >134/>256 |

### Conclusion

The mixed Arg/ Lys type monomer containing peptoids (1-4) have been shown to have anti-bacterial properties against both Gram positive and Gram negative bacteria.

### Example 4 - Biological data: biofilm data

The inventors have demonstrated that the mixed peptoids prepared as described above exhibit surprising antibacterial and antifungal properties against bacterial and fungal biofilms.

### Materials and methods

### Micro-organism strains and growth conditions

*C. albicans* (NCTC 3179) was subcultured aerobically on Sabouraud agar plates and propagated in yeast peptone dextrose broth. *E.coli* (ATCC 29522) and *S.aureus* (NCTC 6571) were grown on blood agar plates and propagated in brain heart infusion (BHI) broth.

### Preparation and treatment of single species biofilms

Overnight cultures of *C*. *albicans* were washed and resuspended in a modified RPMI-1640 (Sigma-Aldrich, St Louis, USA) medium to yield an inoculum of 1.0 × 10⁶ cells/ml. Overnight cultures of *S. aureus* or *E. coli* were washed and resuspended in brain heart infusion broth (Oxoid, Basingstoke, UK) to yield an inoculum of 5.0 × 10⁶ cells/ml. A total volume of 100µl of each inoculum was added to microtitre plate wells (Thermo Fisher Scientific, Roskilde, Denmark). An initial biofilm was allowed to form for 4 hours. Wells were washed three times with 200 µl PBS to facilitate removal of planktonic cells and the biofilms were then treated with 100 µM of peptoids 1-4 in the appropriate broth. Plates were incubated for a further 24 hours to allow biofilm maturation. After removal of planktonic cells by washing, biofilms were quantified by the crystal violet assay or by PMA-qPCR.

### Biofilm quantification by crystal violet assay

Washed biofilms were fixed with 100 µl methanol for 10 minutes. Following removal of methanol, the wells were air dried and stained with crystal violet solution (Clin-Tech Ltd, Guildford, UK) for 20 minutes at room temperature. Excess stain was removed by washing, the plate was then air dried and bound crystal violet was re-solubilised in 160µl 33% acetic acid prior to reading at 570nm in a microtitre plate reader (Tecan GENios, Zürich, Switzerland).

### Results

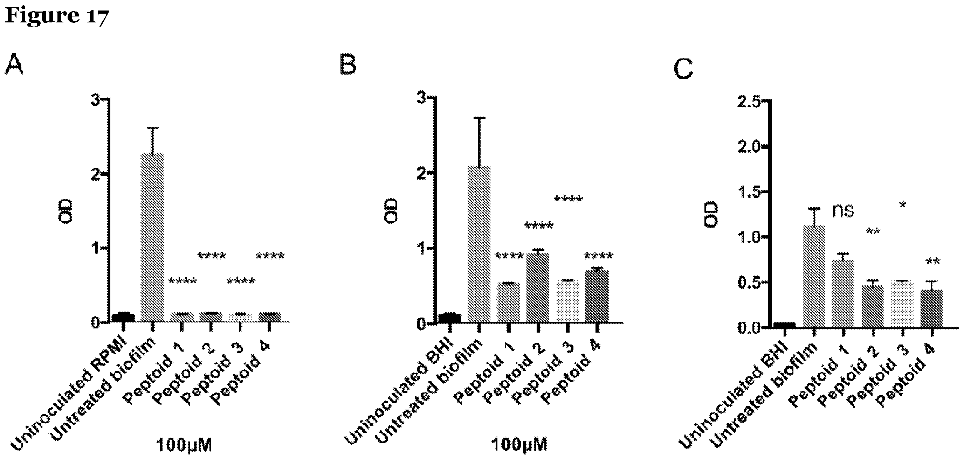

As shown in Figure 17A, all of the peptoids 1 to 4 exhibited strong antifungal activity against the C. *albicans* biofilm. Furthermore, as shown in Figure 17B, all of peptoids 1 to 4 exhibited strong anti-bacterial activity against the *S. aureus* biofilm, with peptoids 1 and 3 exhibiting the strongest anti-bacterial activity. Finally, as shown in Figure 17C, all of peptoids 1 to 4 exhibited anti-bacterial activity against the *E. coli* biofilm, with peptoids 2 and 4 exhibiting the strongest anti-bacterial activity

### Conclusion

The mixed Arg/ Lys type monomer containing peptoids (1-4) have been shown to have antimicrobial properties against fungus (C. *albicans*), gram positive bacteria (*S. aureus*) and gram negative bacteria (*E. coli*).

### Example 5 - Biological data: anti-parasitic activity

The inventors have demonstrated that the mixed peptoids prepared as described above exhibit surprising antiparasitic activity against clinically relevant parasites that cause various diseases; malaria, Chagas disease, African sleeping sickness and Leishmaniasis.

### Materials and methods

### Activity against Trypanosoma brucei rhodesiense STIB900.

This stock was isolated in 1982 from a human patient in Tanzania and after several mouse passages cloned and adapted to axenic culture conditions. Minimum Essential Medium (50 µl) supplemented with 25 mM HEPES, 1g/l additional glucose, 1% MEM non-essential amino acids (100x), 0.2 mM 2-mercaptoethanol, imM Na-pyruvate and 15% heat inactivated horse serum was added to each well of a 96-well microtiter plate. Serial drug dilutions of eleven 3-fold dilution steps covering a range from 100 to 0.002 µg/ml were prepared. Then 4×10³ bloodstream forms of *T. b. rhodesiense* STIB 900 in 50 |ul was added to each well and the plate incubated at 37 °C under a 5 % CO₂ atmosphere for 70 h. 10 µl Alamar Blue (resazurin, 12.5 mg in 100 ml double-distilled water) was then added to each well and incubation continued for a further 2-4 h. Then the plates were read with a Spectramax Gemini XS microplate fluorometer (Molecular Devices Cooperation, Sunnyvale, CA, USA) using an excitation wave length of 536 nm and an emission wave length of 588 nm. The IC50 values were calculated by linear regression from the sigmoidal dose inhibition curves using SoftmaxPro software (Molecular Devices Cooperation, Sunnyvale, CA, USA). Melarsoprol (Arsobal Sanofi-Aventis, received from WHO) is used as control.

### Activity against T. cruzi.

Rat skeletal myoblasts (L-6 cells) were seeded in 96-well microtitre plates at 2000 cells/well in 100 µL RPMI 1640 medium with 10% FBS and 2 mM 1-glutamine. After 24 h the medium was removed and replaced by 100 µl per well containing 5000 trypomastigote forms of *T. cruzi* Tulahuen strain C2C4 containing the β-galactosidase (Lac Z) gene. After 48 h the medium was removed from the wells and replaced by 100 µl fresh medium with or without a serial drug dilution of eleven 3-fold dilution steps covering a range from 100 to 0.002 µg/ml. After 96 h of incubation the plates were inspected under an inverted microscope to assure growth of the controls and sterility. Then the substrate CPRG/Nonidet (50 µl) was added to all wells. A color reaction developed within 2-6 h and could be read photometrically at 540 nm. Data were analyzed with the graphic programme Softmax Pro (Molecular Devices), which calculated IC₅₀ values by linear regression from the sigmoidal dose inhibition curves. Benznidazole is used as control (IC50 0_{.}5±0_{.}2µg/ml).

### Activity against L. donovani axenic amastigotes

Amastigotes *of L. donovani* strain MHOM/ET/67/L82 were grown in axenic culture at 37 °C in SM medium²⁴ at pH 5.4 supplemented with 10% heat-inactivated fetal bovine serum under an atmosphere of 5% CO₂ in air. One hundred microlitres of culture medium with 10⁵ amastigotes from axenic culture with or without a serial drug dilution were seeded in 96-well microtitre plates. Serial drug dilutions of eleven 3-fold dilution steps covering a range from 90 to 0.002 µg/ml were prepared. After 70 h of incubation the plates were inspected under an inverted microscope to assure growth of the controls and sterile conditions. 10 µl of Alamar Blue (12.5 mg resazurin dissolved in 100 ml distilled water) were then added to each well and the plates incubated for another 2 h. Then the plates were read with a Spectramax Gemini XS microplate fluorometer (Molecular Devices Cooperation, Sunnyvale, CA, USA) using an excitation wave length of 536 nm and an emission wave length of 588 nm. Data were analyzed using the software Softmax Pro (Molecular Devices Cooperation, Sunnyvale, CA, USA). Decrease of fluorescence (= inhibition) was expressed as percentage of the fluorescence of control cultures and plotted against the drug concentrations. From the sigmoidal inhibition curves the IC₅₀ values were calculated.

### Activity against P. falciparum.

In vitro activity against erythrocytic stages of *P.falciparum* was determined using a ³H-hypoxanthine incorporation assay using the drug sensitive NF54 strain (Schipol airport) or the chloroquine and pyrimethamine resistant K1 strain that originate from Thailand and the standard drug chloroquine (Sigma C6628). Compounds were dissolved in DMSO at 10 mg/ml and added to parasite cultures incubated in RPMI 1640 medium without hypoxanthine, supplemented with HEPES (5.94 g/1), NaHCO₃ (2.1 g/l), neomycin (100 U/ml), Albumax^{R} (5 g/l) and washed human red cells A⁺ at 2.5% haematocrit (0.3% parasitaemia). Serial drug dilutions of eleven 3-fold dilution steps covering a range from 100 to 0.002 µg/ml were prepared. The 96-well plates were incubated in a humidified atmosphere at 37 °C; 4% CO₂, 3% O₂, 93% N2. After 48 h 50 µl of ³H-hypoxanthine (=0.5 µCi) was added to each well of the plate. The plates were incubated for a further 24 h under the same conditions. The plates were then harvested with a Betaplate^{™} cell harvester (Wallac, Zurich, Switzerland), and the red blood cells transferred onto a glass fibre filter then washed with distilled water. The dried filters were inserted into a plastic foil with 10 ml of scintillation fluid, and counted in a Betaplate^{™} liquid scintillation counter (Wallac, Zurich, Switzerland). IC₅₀ values were calculated from sigmoidal inhibition curves by linear regression using Microsoft Excel. Chloroquine and artemisinin are used as control.

### Cell Culture of Leishmania Mexicana M379 Promastigotes and Amastigotes

Leishmania mexicana (M379) promastigote parasites were maintained at 26 °C in Schneider's Insect medium (Sigma-Aldrich) supplemented with heat-inactivated foetal bovine sera (FBS, 15%; Biosera Ltd). Cells were counted using a Neubauer Improved Haemocytometer. Promastigotes were transformed into axenic amastigotes by a pH and temperature shift as previously described. A culture of recently transformed (three days) promastigotes in the late log phase was transferred into Schneider's Insect medium supplemented with 20% heat-inactivated FBS (pH 5.5) at 5×105 parasites/mL. After 6 days, the parasites were in the metacyclic stage and used for transformation to amastigote-like forms by transfer in the same medium at 32 °C at 5×105 parasites/mL. After additional 5-7 days, the parasites should be in the amastigote stage and be ready for cytotoxicity studies and infections.

### Cytotoxicity Assays with L. Mexicana M379 Promastigotes and Amastigotes

Cytotoxicity analyses were performed in 96-well plates (Costar, Fisher Scientific) using Alamar Blue (Invitrogen) for cell viability detection as previously described. Promastigote and amastigote L. mexicana were pre-incubated with the compounds in triplicate (5mM stock solutions in DMSO; Amphotericin B was used as a positive control; untreated parasites with DMSO as a negative control) in 50 µl of the corresponding media at 4×106 mL-1 for 1 hour. Afterwards, 40 µl were removed from each well before the addition of 90 µl of the corresponding media, followed by incubation for 24 hours at 4×105 mL-1. Then, 10 µl Alamar Blue solution (Invitrogen) was added to each well for an incubation of 4 hours prior to assessing cell viability using a fluorescent plate reader (Biotek; Ex 560 nm / Em 600 nm). To investigate the effects of serum on the efficacy of the peptoids, the assay described above was modified using serum-free medium for the pre-incubation time. For these assays, the parasites were washed three times in serum-free medium before adding them to the compound solutions. All of the experiments described above were carried out on a minimum of two separate occasions to ensure a robust data set was collected.

### Results

The anti-parasitic activity of peptoids 1, 2, 4 and 6 against biofilm are shown in Table 2.

**Table 2: Anti-parasitic activities of peptoids 1, 2, 4 and 6.**

| **Peptoid** | **IC50 (µM )** | | | | |
|---|---|---|---|---|---|
| | *L. mexicana* | *T. brucei rhodesiense* | *T. cruzi* | *L. donovani* | *P. falciparum* |
| 1 | 37 | 6.73 | 12.35 | 11.14 | 1.50 |
| 2 | 34 | | | | |
| 4 | 37 | 16.70 | 21.50 | 19.95 | 2.72 |
| 6 | | 6.44 | 6.58 | 9.51 | 0.99 |

Peptoids 1, 2 and 4 were all found to be active against *L. mexicana*, which causes cutaneous leishmaniasis. Additionally, peptoids 1, 4 and 6 were all found to be active against *T. brucei rhodesiense*, which causes causes African sleeping sickness, *T. cruzi* which causes Chagas disease, *L. donovani* which causes visceral leishmaniasis, and *P*. *falciparum*, which causes malaria.

### Conclusion

Peptoids 1, 2, 4 and 6 have been shown to have anti-parasitic activities against various protozoan parasites.

## Claims

1. A method of preparing a peptoid, analogue or derivative thereof, comprising at least one arginine type monomer and at least one lysine type monomer, wherein the arginine type monomer comprises a monomer of Formula (I): 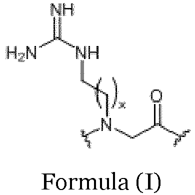 wherein x is an integer between 0 and 14; and
the lysine type monomer comprises a monomer of Formula (II): 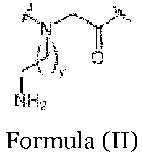 wherein y is an integer between 0 and 14;
the method comprising:-
(i) synthesising a precursor linear peptoid, analogue or derivative thereof comprising one or more lysine type monomers protected with a first protecting group, and one or more lysine type monomers protected with a second protecting group, wherein the first protecting group comprises an N-(1-(4,4-dimethyl-2,6-dioxocyclohexylidene)ethyl) (Dde) protecting group and the second protecting group comprises a tert-butyloxycarbonyl (Boc) protecting group;
(ii) removing the first protecting group to reveal one or more unprotected lysine type monomers;
(iii) converting the one or more unprotected lysine type monomers to one or more arginine type monomers; and
(iv) removing the second protecting group to obtain a peptoid, analogue or derivative thereof, comprising at least one arginine type monomer and at least one lysine type monomer.

2. A method according to either claim 1, wherein the step of synthesising the linear precursor peptoid, analogue or derivative thereof comprises:
- synthesising a first monomer on a substrate to obtain a linear precursor peptoid, analogue or derivative thereof comprising one monomer;
- subsequently adding at least one further monomer in a step-wise fashion to obtain the linear precursor peptoid, analogue or derivative thereof containing the desired number of monomers.

3. A method according to claim 2, wherein the first and/or at least one further monomer comprise at least one monomer comprising an aromatic residue and/or at least one monomer comprising an aliphatic residue, preferably
- wherein the monomer comprising an aromatic residue comprises an (S)-*N*-(1-phenylethyl) glycine (*N*spe) monomer, an (R)-*N*-(1-phenylethyl) glycine (*N*rpe) monomer, an *N*-(phenylmethyl) glycine (Nphe) monomer, an *N*-(4-fluoro phenylmethyl) glycine (*N*pfb) monomer, an *N*-(3-fluoro phenylmethyl) glycine (*N*mfb) monomer, an (S)-*N*-1-(4-fluoro phenylethyl) glycine (*N*sfb) monomer, an (R)-*N*-1-(4-fluoro phenylethyl) glycine (*N*rfb) monomer, an *N*-(3,5 difluoro phenylmethyl) glycine (*N*dfb) monomer, an *N*-(4-chloro phenylmethyl) glycine (*N*pcb) monomer, an *N*-(4-methoxyphenylmethyl) glycine (*N*pmb) monomer, an *N*-(methylimidazole) glycine (*N*His) monomer, an *N*-(methylindole) glycine (*N*Trp) monomer, an *N*-(4-hydroxy phenylmethyl) glycine (*N*Tyr) monomer, an *N*-(4-pyridinylmethyl) glycine (*N*Pyr) monomer, an (S)-*N*-(1-naphthlethyl) glycine (*N*sna) monomer, an (R)-*N*-(1-naphthlethyl) glycine (*N*rna) monomer, an *N*-(furanylmethyl) glycine (*N*fur) monomer, an *N*-(thiofuranylmethyl) glycine (*N*tfur) monomer, or an *N-*(diphenylmethyl) glycine (*N*dpa) monomer; and/or
- wherein the monomer comprising an aliphatic residue comprises an *N*-(pentyl) glycine (*N*amy) monomer, an *N*-(propyl) glycine (*N*NVa) monomer, an *N*-(isopentyl) glycine (*N*HLe) monomer, *N*-(isobutyl) glycine (*N*Leu) monomer, an *N*-(butyl) glycine (*N*but) monomer, an *N*-(2-carboxyethyl) glycine (*N*Glu) monomer, an *N*-(2,2,2-trifluoromethyl) glycine (*N*tfe) monomer, an *N*-(2,2,3,3,3-pentafluoropropyl) glycine (*N*pfp), an *N*-(2,2 - difluoroethyl) glycine (*N*dfea) monomer, an *N*-(ethyl) glycine (Nea) monomer, an *N*-(2-thioethyl) glycine (NCys) monomer, an (*S*)-*N*-(sec-butyl) glycine (*N*ssb) monomer, an (*R*)*-N-*(sec-butyl) glycine (Nrsb) monomer, an (*S*)-*N*-(1-methylbutyl) glycine (*N*smb) monomer, an (R)-*N*-(1-methylbutyl) glycine (*N*rmb) monomer, an (S)-*N*-(1-cyclohexylethyl) glycine (*N*sch) monomer, (*R*)-*N*-(1-cyclohexylethyl) glycine (*N*rch) monomer, an *N*-(1-cyclohexylmethyl) glycine (*N*ch) monomer, an *N*-(ethynylmethyl) glycine (*N*em) monomer, an (*S*)-*N*-(1-ethynylethyl) glycine (Nsee) monomer, or an (*R*)-*N*-(1-ethynylethyl) glycine (*N*ree) monomer.

4. A method according to claim 3, wherein the monomer comprising an aromatic residue comprises an (*S*)-*N*-(1-phenylethyl) glycine (*N*spe) monomer.

5. A method according any one of claims 2 to 4, wherein the substrate comprises a resin, preferably Rink amide resin, 2-chlorotrityl chloride resin, Wang resin, 4-(1',1'-dimethyl-1'-hydroxypropyl) phenoxyacetyl alanyl aminomethyl polystyrene (DHPP) resin or diphenyldiazomethane (PDDM) resin.

6. A method according to any one of claims 2 to 5, wherein the method comprises a step of cleaving the peptoid, derivative or analogue thereof from the substrate to obtain a cleaved precursor linear peptoid, analogue or derivative thereof, preferably wherein:
- the cleaving step is carried out subsequent to step (i) and prior to step (ii), more preferably wherein the method comprises a cyclisation step comprising cyclising the precursor linear peptoid, derivative or analogue thereof to obtain a precursor cyclic peptoid, wherein the cyclisation step is carried out subsequent to the cleaving step, and prior to step (ii); or
- the cleaving step is carried out subsequent to step (iii), more preferably wherein the cleaving step is carried out simultaneously to the step of removing the second protecting group.

7. A peptoid, analogue or derivative thereof wherein the peptoid, analogue or derivative thereof comprises:
- a linear peptoid which has the structure (*N*Lys-*N*spe-*N*spe)₂(*N*hArg-*N*spe-*N*spe)₂; (*N*hArg-*N*spe-*N*spe)₂(*N*Lys-*N*spe-*N*spe)₂; (*N*Lys-*N*spe-*N*spe)(*N*hArg-*N*spe-*N*spe)(*N*Lys-*N*spe-*N*spe)₂; [(*N*hArg-*N*spe-*N*spe)(*N*Lys-*N*spe-*N*spe)]₂; or [(*N*nArg*N*spe*N*spe)(*N*ae*N*spe*N*spe)]₂; or
- a cyclic peptoid which has the structure (*N*Lys-*N*phe-*N*hArg-*N*phe-*N*Lys-*N*phe).

8. A peptoid, analogue or derivative thereof according to claim 7, for use as a medicament.

9. A peptoid, analogue or derivative thereof according to claim 7, for use in treating, ameliorating or preventing a microbial infection in a vertebrate.

10. A peptoid, analogue or derivative thereof for use according to claim 9, wherein the microbial infection comprises a bacterial infection, a fungal infection or a parasitic infection.

11. A peptoid, analogue or derivative thereof for use according to claim 10, wherein:
- the bacterial infection comprises a gram positive bacterial infection or a gram negative bacterial infection, and/or wherein the bacterium is from the *Escherichia* genus, preferably *E. coli, Pseudomonas* genus, preferably P. *aeruginosa, Staphylococcus* genus, preferably *S. aureus,* or *Serratia* genus, preferably *S. marcesens;* and/or
- the fungus is from the *Candida* genus, preferably *C*. *albicans;* and/or
- the parasite is from the *Leishmania* genus, preferably *L. mexicana* or *L. donovani,* the *Trypanosoma* genus, preferably *T. brucei* or *T. cruzi,* the *Plasmodium* genus, preferably *P. falciparum,* and/or the peptoid, analogue or derivative thereof is for use in treating, ameliorating or preventing leishmaniasis, African trypanosomiasis, Chagas disease or malaria.

12. A pharmaceutical composition comprising a peptoid, analogue or derivative thereof according to claim 7, and a pharmaceutically acceptable vehicle.

## Patentansprüche

1. Verfahren zum Herstellen eines Peptoids, Analogons oder Derivats davon, das mindestens ein Monomer vom Arginintyp und mindestens ein Monomer vom Lysintyp umfasst, wobei das Monomer vom Arginintyp ein Monomer der Formel (I) umfasst: 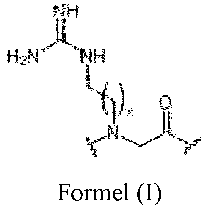 wobei x eine ganze Zahl zwischen 0 und 14 ist; und
das Monomer vom Lysintyp ein Monomer der Formel (II) umfasst: 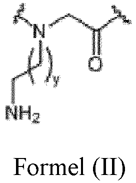 wobei y eine ganze Zahl zwischen 0 und 14 ist;
wobei das Verfahren umfasst:-
(i) Synthetisieren eines linearen Vorläufer-Peptoids, -Analogons oder -Derivats davon, das ein oder mehrere Monomere vom Lysintyp, die mit einer ersten Schutzgruppe geschützt sind, und ein oder mehrere Monomere vom Lysintyp, die mit einer zweiten Schutzgruppe geschützt sind, umfasst, wobei die erste Schutzgruppe eine N-(1-(4,4-Dimethyl-2,6-dioxocyclohexyliden)ethyl)(Dde)-Schutzgruppe umfasst und die zweite Schutzgruppe eine tert-Butyloxycarbonyl (Boc)-Schutzgruppe umfasst;
(ii) Entfernen der ersten Schutzgruppe, um ein oder mehrere ungeschützte Monomere vom Lysintyp freizulegen;
(iii) Umwandeln des einen oder der mehreren ungeschützten Monomere vom Lysintyp in ein oder mehrere Monomere vom Arginintyp; und
(iv) Entfernen der zweiten Schutzgruppe, um ein Peptoid, Analogon oder Derivat davon zu erhalten, das mindestens ein Monomer vom Arginintyp und mindestens ein Monomer vom Lysintyp umfasst.

2. Verfahren nach Anspruch 1, wobei der Schritt des Synthetisierens des linearen Vorläufer-Peptoids, -Analogons oder -Derivats davon umfasst:
- Synthetisieren eines ersten Monomers auf einem Substrat, um ein lineares Vorläufer-Peptoid, -Analogon oder -Derivat davon zu erhalten, das ein Monomer umfasst;
- anschließend schrittweises Zugeben mindestens eines weiteren Monomers, um das lineare Vorläufer-Peptoid, -Analogon oder -Derivat davon zu erhalten, das die gewünschte Anzahl an Monomeren enthält.

3. Verfahren nach Anspruch 2, wobei das erste und/oder mindestens eine weitere Monomer mindestens ein Monomer umfasst, das einen aromatischen Rest umfasst, und/oder mindestens ein Monomer umfasst, das einen aliphatischen Rest umfasst, bevorzugt
- wobei das Monomer, das einen aromatischen Rest umfasst, ein (*S*)-*N*-(1-Phenylethyl) glycin(*N*spe)-Monomer, ein (*R*)-*N*-(1-Phenylethyl)glycin(*N*rpe)-Monomer, ein *N-*(Phenylmethyl)glycin(*N*phe)-Monomer, ein *N*-(4-Fluorphenylmethyl)glycin(*N*pfb)-Monomer, ein *N*-(3-Fluorphenylmethyl)glycin(*N*mfb)-Monomer, ein (*S*)-*N*-1-(4-Fluorphenylethyl)glycin(*N*sfb)-Monomer, ein (*R*)-*N*-1-(4-Fluorphenylethyl)glycin(*N*rfb)-Monomer, ein *N*-(3,5-Difluorphenylmethyl)glycin(*N*dfb)-Monomer, ein *N*-(4-Chlorphenylmethyl)glycin(*N*pcb)-Monomer, ein *N*-(4-Methoxyphenylmethyl)glycin(*N*pmb)-Monomer, ein *N-*(Methylimidazol)glycin(*N*His)-Monomer, ein *N*-(Methylindol)glycin(*N*Trp)-Monomer, ein *N*-(4-Hydroxyphenylmethyl)glycin(*N*Tyr)-Monomer, ein *N*-(4-Pyridinylmethyl)glycin(*N*Pyr)-Monomer, ein (*S*)-*N*(1-Naphthlethyl)glycin(*N*sna)-Monomer, ein (*R*)*-N-(*1-Naphthlethyl)glycin(*N*rna)-Monomer, ein *N*-(Furanylmethyl)glycin(*N*tfur)-Monomer, ein *N-*(Thiofuranylmethyl)glycin(*N*tfur)-Monomer oder ein *N*-(Diphenylmethyl)glycin(*N*dpa)-Monomer umfasst; und/oder
- wobei das Monomer, das einen aliphatischen Rest umfasst, ein *N*-(Pentyl)glycin(*N*amy)-Monomer, ein *N*-(Propyl)glycin (*N*NVa)-Monomer, ein *N*-(Isopentyl)glycin(*N*HLe)-Monomer, *N-*(Isobutyl)glycin(*N*Leu)-Monomer, ein *N*-(Butyl)glycin (*N*but)-Monomer, ein *N-*(2-Carboxyethyl)glycin(*N*Glu)-Monomer, ein *N*-(2,2,2-Trifluoromethyl)glycin(*N*tfe)-Monomer, ein N-(2,2,3,3,3-Pentafluorpropyl)glycin(*N*pfp), ein *N*-(2,2-Difluorethyl)glycin(*N*dfea)-Monomer, ein *N*-(Ethyl)glycin(*N*ea)-Monomer, ein *N*-(2-Thioethyl)glycin (*N*Cys)-Monomer, ein (*S*)-*N*-(sec-Butyl)glycin(*N*ssb)-Monomer, ein (*R*)-*N*-(sec-Butyl)glycin(*N*rsb)-Monomer, ein (*S*)-*N*-(1-Methylbutyl)glycin(*N*smb)-Monomer, ein (*R*)-*N*-(1-Methylbutyl)glycin(*N*rmb)-Monomer, ein *(S)-N-(1*-Cyclohexylethyl)glycin(*N*sch)-Monomer, (*R*)-*N*-(1-Cyclohexylethyl)glycin(*N*rch)-Monomer, ein *N*-(1-Cyclohexylmethyl)glycin(Arch)-Monomer, ein *N-*(Ethynylmethyl)glycin(*N*em)-Monomer, ein (S)-*N*-(1-Ethynylethyl)glycin(*N*see)-Monomer oder ein *(R)*-*N*-(1-Ethynylethyl)glycin(*N*ree)-Monomer umfasst.

4. Verfahren nach Anspruch 3, wobei das Monomer, das einen aromatischen Rest umfasst, ein (S)-*N*-(1-Phenylethyl)glycin(*N*spe)-Monomer umfasst.

5. Verfahren nach einem der Ansprüche 2 bis 4, wobei das Substrat ein Harz, bevorzugt Rink-Amid-Harz, 2-Chlortritylchlorid-Harz, Wang-Harz, 4-(1',1'-Dimethyl-1'-hydroxypropyl)phenoxyacetylalanylaminomethylpolystyrol(DHPP)-Harz oder Diphenyldiazomethan(PDDM)-Harz umfasst.

6. Verfahren nach einem der Ansprüche 2 bis 5, wobei das Verfahren einen Schritt eines Spaltens des Peptoids, Derivats oder Analogons davon von dem Substrat umfasst, um ein gespaltenes lineares Vorläufer-Peptoid, -Analogon oder -Derivat davon zu erhalten, wobei bevorzugt:
- der Schritt des Spaltens nach Schritt (i) und vor Schritt (ii) durchgeführt wird, besonders bevorzugt wobei das Verfahren einen Schritt eines Cyclisierens umfasst, der ein Cyclisieren des linearen Vorläufer-Peptoids, -Derivats oder -Analogons davon umfasst, um ein zyklisches Vorläufer-Peptoid zu erhalten, wobei der Schritt der Cyclisierung nach dem Schritt des Spaltens und vor Schritt (ii) durchgeführt wird; oder
- der Schritt des Spaltens nach Schritt (iii) durchgeführt wird, wobei besonders bevorzugt der Schritt des Spaltens gleichzeitig mit dem Schritt des Entfernens der zweiten Schutzgruppe ausgeführt wird.

7. Peptoid, Analogon oder Derivat davon, wobei das Peptoid, Analogon oder Derivat davon umfasst:
- ein lineares Peptoid, das die Struktur (*N*Lys-*N*spe-*N*spe)₂(*N*hArg-*N*spe-*N*spe)₂; (*N*hArg-*N*spe-*N*spe)₂(*N*Lys-*N*spe-*N*spe)₂;(*N*Lys-*N*spe-*N*spe)(*N*Arg-*N*spe-*N*spe)(*N*Lys-*N*spe-*N*spe)₂; [(*N*hArg-*N*spe-*N*spe)(*N*Lys-*N*spe-*N*spe)]₂; oder [(*N*nArg*N*spe*N*spe)(*N*ae*N*spre*N*spe)]₂ aufweist; oder
- ein zyklisches Peptoid, das die Struktur (*N*Lys-*N*phe-*N*hArg-*N*phe-*N*Lys-*N*phe) aufweist.

8. Peptoid, Analogon oder Derivat davon nach Anspruch 7 zur Verwendung als Medikament.

9. Peptoid, Analogon oder Derivat davon nach Anspruch 7 zur Verwendung beim Behandeln, Verbessern oder Vorbeugen einer mikrobiellen Infektion bei einem Wirbeltier.

10. Peptoid, Analogon oder Derivat davon zur Verwendung nach Anspruch 9, wobei die mikrobielle Infektion eine bakterielle Infektion, eine Pilzinfektion oder eine parasitäre Infektion umfasst.

11. Peptoid, Analogon oder Derivat davon zur Verwendung nach Anspruch 10, wobei:
- die bakterielle Infektion eine grampositive bakterielle Infektion oder eine gramnegative bakterielle Infektion umfasst und/oder wobei das Bakterium von der Gattung *Escherichia,* bevorzugt *E. coli,* Gattung *Pseudomonas,* bevorzugt *P. aeruginosa,* Gattung *Staphylococcus,* bevorzugt *S. aureus,* oder Gattung *Serratia,* bevorzugt S. *marcesens* stammt; und/oder
- der Pilz von der Gattung *Candida,* bevorzugt C. *albicans* stammt; und/oder
- der Parasit von der Gattung *Leishmania,* bevorzugt *L. mexicana* oder *L. donovani,* der Gattung *Trypanosoma,* bevorzugt *T. brucei* oder *T. cruzi,* der Gattung *Plasmodium,* bevorzugt *P*. *falciparum* stammt, und/oder das Peptoid, Analogon oder Derivat davon zur Verwendung beim Behandeln, Verbessern oder Vorbeugen von Leishmaniose, afrikanischer Trypanosomiasis, Chagas-Krankheit oder Malaria dient.

12. Pharmazeutische Zusammensetzung, umfassend ein Peptoid, Analogon oder Derivat davon nach Anspruch 7 und ein pharmazeutisch unbedenkliches Vehikel.

## Revendications

1. Procédé de préparation d'un peptoïde ou d'un analogue ou dérivé de celui-ci, comprenant au moins un monomère de type arginine et au moins un monomère de type lysine, où le monomère de type arginine comprend un monomère de Formule (I) : 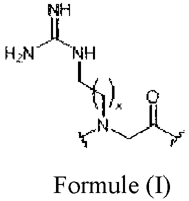 où x est un nombre entier entre 0 et 14 ; et
le monomère de type lysine comprend un monomère de Formule (II) : 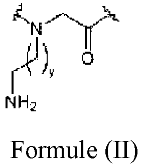 où y est un nombre entier entre 0 et 14 ;
le procédé comprenant :-
(i) la synthétisation d'un peptoïde linéaire précurseur ou d'un analogue ou dérivé de celui-ci, comprenant un ou plusieurs monomères de type lysine protégés par un premier groupe protecteur, et un ou plusieurs monomères de type lysine protégés par un deuxième groupe protecteur, où le premier groupe protecteur comprend un groupe protecteur N-(1-(4,4-diméthyl-2,6-dioxocyclohexylidène)éthyle) (Dde) et le deuxième groupe protecteur comprend un groupe protecteur tert-butyloxycarbonyle (Boc) ;
(ii) l'enlèvement du premier groupe protecteur pour révéler un ou plusieurs monomères de type lysine non protégés ;
(iii) la conversion du ou des monomères de type lysine non protégés en un ou plusieurs monomères de type arginine ; et
(iv) l'enlèvement du deuxième groupe protecteur pour obtenir un peptoïde ou un analogue ou dérivé de celui-ci, comprenant au moins un monomère de type arginine et au moins un monomère de type lysine.

2. Procédé selon la revendication 1, dans lequel l'étape de synthétisation du peptoïde précurseur linéaire ou d'un analogue ou dérivé de celui-ci comprend :
- la synthétisation d'un premier monomère sur un substrat pour obtenir un peptoïde précurseur linéaire ou un analogue ou dérivé de celui-ci comprenant un monomère ;
- suivie de l'adjonction d'au moins un autre monomère de manière progressive pour obtenir le peptoïde précurseur linéaire ou un analogue ou dérivé de celui-ci contenant le nombre voulu de monomères.

3. Procédé selon la revendication 2, dans lequel le premier et/ou au moins un autre monomère comprennent au moins un monomère comprenant un résidu aromatique et/ou au moins un monomère comprenant un résidu aliphatique, de préférence
- où le monomère comprenant un résidu aromatique comprend un monomère (S)-*N*-(1-phényléthyl) glycine (*N*spe), un monomère (R)-*N*-(1-phényléthyl) glycine (*N*rpe), un monomère *N*-(phénylméthyl) glycine (*N*phe), un monomère *N*-(4-fluorophénylméthyl) glycine (*N*pfb), un monomère *N*-(3-fluorophénylméthyl) glycine (*N*mfb), un monomère (S)-*N*-1-(4-fluoro phényléthyl) glycine (*N*sfb), un monomère (*R*)-*N*-1-(4-fluorophényléthyl) glycine (*N*rfb), un monomère *N-(3,5* difluorophénylméthyl) glycine (*N*dfb), un monomère *N*-(4-chlorophénylméthyl) glycine (*N*pcb), un monomère *N*-(4-méthoxyphénylméthyl) glycine (*N*pmb), un monomère *N*-(méthylimidazole) glycine (*N*His), un monomère *N*-(méthylindole) glycine (*N*Trp), un monomère *N*-(4-hydroxyphénylméthyl) glycine (*N*Tyr), un monomère *N*-(4-pyridinylméthyl) glycine (*N*Pyr), un monomère (*S*)-*N*-(1-naphthyléthyl) glycine (*N*sna), un monomère (*R*)-*N*-(1-naphthyléthyl) glycine (*N*rna), un monomère *N*-(furanylméthyl) glycine (*N*tfur), un monomère *N*-(thiofuranylméthyl) glycine (*N*tfur), ou un monomère *N-*(diphénylméthyl) glycine (*N*dpa) ; et/ou
- où le monomère comprenant un résidu aliphatique comprend un monomère *N*-(pentyl) glycine (*N*amy), un monomère *N*-(propyl) glycine (*N*NVa), un monomère *N*-(isopentyl) glycine (*N*HLe), un monomère *N*-(isobutyl) glycine (*N*Leu), un monomère *N*-(butyl) glycine (*N*but), un monomère *N*-(2-carboxyéthyl) glycine (*N*Glu), un monomère *N*-(2,2,2-trifluorométhyl) glycine (*N*tfe), un *N*-(2,2,3,3,3-pentafluoropropyl) glycine (*N*pfp), un monomère *N*-(2,2-difluoroéthyl) glycine (*N*dfea), un monomère *N*-(éthyl) glycine (*N*ea), un monomère *N*-(2-thioéthyl) glycine *(NCys),* un monomère (S)-*N*-(sec-butyl) glycine (*N*ssb), un monomère (*R)*-*N*-(*sec*-butyl) glycine (*N*rsb), un monomère (S)-*N*-(1 -méthylbutyl) glycine (*N*smb), un monomère (*R*)-*N*-(1-méthylbutyl) glycine (*N*rmb), un monomère (*S*)-*N*-(1-cyclohexyléthyl) glycine (*N*sch), un monomère (*R*)-*N*-(1-cyclohexyléthyl) glycine (*N*rch), un monomère *N*-(1-cyclohexylméthyl) glycine (*N*ch), un monomère *N*-(éthynylméthyl) glycine (*N*em), un monomère (S)-*N*-(1-éthynyléthyl) glycine (*N*see), ou un monomère (*R*)-*N*-(1-éthynyléthyl) glycine (*N*ree).

4. Procédé selon la revendication 3, dans lequel le monomère comprenant un résidu aromatique comprend un monomère (S)-*N*-(1-phényléthyl) glycine (*N*spe).

5. Procédé selon l'une quelconque des revendications 2 à 4, dans lequel le substrat comprend une résine, de préférence une résine amide de Rink, résine de chlorure de 2-chlorotrityle, résine de Wang, résine de 4-(1',1'-diméthyl-1'-hydroxypropyl) phénoxyacétyl alanyl aminométhyl polystyrène (DHPP) ou résine de diphényldiazométhane (PDDM).

6. Procédé selon l'une quelconque des revendications 2 à 5, le procédé comprenant une étape de clivage du peptoïde, de son dérivé ou de son analogue, du substrat pour obtenir un peptoïde linéaire précurseur clivé ou un analogue ou dérivé de celui-ci, de préférence dans lequel :
- l'étape de clivage est effectuée à la suite de l'étape (i) et avant l'étape (ii), de préférence où le procédé comprend une étape de cyclisation comprenant la cyclisation du peptoïde linéaire précurseur ou du dérivé ou analogue de celui-ci pour obtenir un peptoïde cyclique précurseur, où l'étape de cyclisation est effectuée à la suite de l'étape de clivage et avant l'étape (ii) ; ou
- l'étape de clivage est effectuée à la suite de l'étape (iii), de préférence où l'étape de clivage est effectuée simultanément à l'étape d'enlèvement du deuxième groupe protecteur.

7. Peptoïde ou analogue ou dérivé de celui-ci, où le peptoïde, son analogue ou son dérivé comprend :
- un peptoïde linéaire qui a la structure (*N*Lys-*N*spe-Nspe)₂(*N*hArg-*N*spe-*N*spe)₂ ; (*N*hArg-*N*spe-*N*spe)₂(*N*Lys-*N*spe-*N*spe)₂ ; (*N*Lys-*N*spe-*N*spe)(*N*hArg-*N*spe-*N*spe)(*N*Lys-*N*spe-*N*spe)₂;[(*N*hArg-*N*spe-*N*spe)(*N*Lys-*N*spe-*N*sp e)]₂; ou [(*N*nArg*N*spe*N*spe)(*N*ae*N*spre*N*spe)]₂; ou
- un peptoïde cyclique qui a la structure (*N*Lys-*N*phe-*N*hArg-*N*phe-*N*Lys-*N*phe).

8. Peptoïde, ou analogue ou dérivé de celui-ci selon la revendication 7, pour utilisation comme médicament.

9. Peptoïde, ou analogue ou dérivé de celui-ci selon la revendication 7, pour utilisation dans le traitement, l'amélioration ou la prévention d'une infection microbienne chez un vertébré.

10. Peptoïde, ou analogue ou dérivé de celui-ci pour utilisation selon la revendication 9, où l'infection microbienne comprend une infection bactérienne, une infection fongique ou une infection parasitaire.

11. Peptoïde, ou analogue ou dérivé de celui-ci, pour utilisation selon la revendication 10, où :
- l'infection bactérienne comprend une infection bactérienne gram positif ou une infection bactérienne gram négatif, et/ou la bactérie est du genre *Escherichia,* de préférence du genre *E*. *coli, Pseudomonas,* de préférence du genre *P. aeruginosa, Staphylococcus,* de préférence du genre *S*. *aureus,* ou *Serratia,* de préférence *S. marcesens ;* et/ou
- le champignon est du genre *Candida,* de préférence *C*. *albicans ;* et/ou
- le parasite est du genre *Leishmania,* de préférence *L. mexicana* ou *L. donovani,* du genre *Trypanosoma,* de préférence *T. brucei* ou *T. cruzi,* du genre *Plasmodium,* de préférence P. *falciparum,* et/ou le peptoïde, ou l'analogue ou le dérivé de celui-ci est prévu pour l'utilisation dans le traitement, l'amélioration ou la prévention de la leishmaniose, de la trypanosomiase africaine, de la maladie de Chagas ou de la malaria.

12. Composition pharmaceutique comprenant un peptoïde, ou un analogue ou dérivé de celui-ci selon la revendication 7, et un excipient de qualité pharmaceutique.
